# FASCICULE DE BREVET EUROPEEN

(11) **EP 1 678 175 B1**
(45) Date de publication et mention de la délivrance du brevet: **13.02.2008**
(21) Numéro de dépôt: 04805771.5
(22) Date de dépôt: 21.10.2004
(51) Int. Cl.: C07D 471/04, C07D 513/04, A61P 1/12, A61P 11/08, A61K 31/519, A61K 31/706

(54) **UTILISATION MEDICALE DE COMPOSES ZWITTERIONIQUES COMME MODULATEURS DES CANAUX CFTR**
MEDIZINISCHE VERWENDUNG VON ZWITTERION-VERBINDUNGEN ALS MODULATOREN DES CFTR -KANALS
MEDICAL USE OF ZWITTERIONIC COMPOUNDS BEING CFTR CHANNEL MODULATORS

(30) Priorité: 23.10.2003 FR 0312417
(43) Date de publication de la demande: 12.07.2006
(73) Titulaire: UNIVERSITE JOSEPH FOURIER, 38040 Grenoble (FR); CENTRE NATIONAL DE LA RECHERCHE SCIENTIFIQUE (CNRS), 75794 Paris Cedex 16 (FR); UNIVERSITE DE POITIERS, 86034 Poitiers Cedex (FR)
(72) Inventeur: DECOUT, Jean-Luc, F-38410 Vaulnaveys le Haut (FR); ROUTABOUL, Christel, F-38400 Saint Martin d'Heres (FR); BECQ, Frédéric, F-86280 Saint Benoit (FR); NOREZ, Caroline, F-86000 Poitiers (FR)
(74) Mandataire: Vuillermoz, Bruno
(86) Numéro de dépôt international: PCT/FR2004/050528
(87) Numéro de publication internationale: WO 2005/039589

(56) Documents cités:
- MA TONGHUI ET AL: "High-affinity Activators of Cystic Fibrosis Transmembrane Conductance Regulator (CFTR) Chloride Conductance Identified by High-Throughput Screening" JOURNAL OF BIOLOGICAL CHEMISTRY, AMERICAN SOCIETY OF BIOLOGICAL CHEMISTS, BALTIMORE, MD, US, vol. 277, no. 40, 4 octobre 2002 (2002-10-04), pages 37235-37241, XP002276764 ISSN: 0021-9258 cité dans la demande
- AL-AWQATI QAIS: "Alternative treatment for secretory diarrhea revealed in a new class of CFTR inhibitors." THE JOURNAL OF CLINICAL INVESTIGATION. DEC 2002, vol. 110, no. 11, décembre 2002 (2002-12), pages 1599-1601, XP009046415 ISSN: 0021-9738 cité dans la demande
- MA TONGHUI ET AL: "Thiazolidinone CFTR inhibitor identified by high-throughput screening blocks cholera toxin-induced intestinal fluid secretion." JOURNAL OF CLINICAL INVESTIGATION, vol. 110, no. 11, décembre 2002 (2002-12), pages 1651-1658, XP002324654 ISSN: 0021-9738 cité dans la demande

## Description

L'invention concerne des compositions pharmaceutiques destinées au traitement de maladies associées à un dysfonctionnement des canaux CFTR, telles que notamment mucoviscidose, asthme ou diarrhée. Ces compositions contiennent une molécule, se présentant à pH physiologique sous la forme d'un zwittérion, de formule : 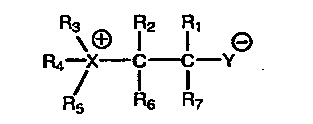 avec X = N ou P ;
Y = O ou S ;
R₁ à R₇ représentent: ⁻, H, ou des chaînes carbonées, substituées ou non, pouvant contenir des hétéroatomes ;
à l'exception de la bétaïne.

La protéine CFTR ou "cystic fibrosis transmembrane conductance regulator" est une protéine trans-membranaire qui a été mise en évidence dans le cadre de la recherche du gène responsable de la mucoviscidose (ou fibrose kystique). Le canal CFTR apparaît être une des clefs de la régulation du transport d'ions chlorure dans les cellules épithéliales. Les épithéliums forment une barrière continue entre l'environnement extérieur et le milieu interne tout en permettant le transport d'ions, de solutés et de macromolécules entre ces compartiments. L'absorption de Na⁺ et la sécrétion de Cl⁻ sont des éléments majeurs de la fonction épithéliale.

Les ions chlorure pénètrent dans la cellule épithéliale par la surface basolatérale, par l'intermédiaire d'un co-transporteur, sous forme (Na⁺, K⁺, 2 Cl⁻) et en sortent en utilisant les canaux CFTR de la surface apicale [1].

Deux types de pathologie peuvent être associés à des dysfonctionnements opposés des canaux CFTR.

Une inhibition du fonctionnement est responsable des problèmes liés à la mucoviscidose. Dans cette maladie, en raison de mutations génétiques, soit la protéine CFTR encodée n'est pas délivrée à la surface apicale, soit elle ne peut pas être activée, d'où une impossibilité pour les ions chlorure de sortir de la cellule épithéliale. Ce phénomène induit une absorption des ions sodium et se traduit par un épaississement du mucus, obstruant les voies respiratoires.

Inversement, une suractivité du canal CFTR est à l'origine de diarrhées. L'élimination excessive de sels s'accompagne d'une perte d'eau, donc de problèmes liés à cette déshydratation. L'exaltation du fonctionnement des canaux CFTR peut être consécutive à la présence de toxines de bactéries, telles que *Escherichia coli* ou *Vibrio cholerae*.

La protéine CFTR fait partie de la superfamille des transporteurs ABC (ATP-Binding Cassettes). Elle est constituée de 1480 acides aminés [**2, 3, 4**] composant deux parties homologues reliées entre elles par un domaine régulateur cytoplasmique (R). Chaque partie comporte six domaines transmembranaires (M1 à M6 et M7 à M12) et un domaine de fixation de nucléotides (NBD-1 et NBD-2). Entre les domaines transmembranaires M7 et M8 sont présents deux sites *N*-glycosylés.

Les douze domaines transmembranaires forment un canal dont l'activité est déterminée par la phosphorylation du domaine R, ainsi que par l'hydrolyse de molécules d'ATP fixées dans les NBDs. Dans le cône extracellulaire sont présents anions et cations. La sélectivité de ce canal pour les anions serait principalement due à la présence d'un résidu arginine Arg-352 (R352) à l'extrémité du cône cytoplamisque. Le diamètre minimum de ce pore serait d'environ 5,3 Å. Cette taille a été déterminée à partir des anions les plus gros pénétrant dans la cellule. Toutefois, de manière transitoire, le canal pourrait se dilater jusqu'à 13 Å de diamètre.

Les différents activateurs des canaux CFTR, connus à ce jour, agissent de différentes manières. On peut citer entre autres trois modes d'action :
- ceux qui agissent directement sur les NBD : la génistéine qui prolonge l'ouverture du canal; le NS-004 ou les MPB (benzo[c]quinolizinium substitués) ;
- ceux qui agissent par augmentation de la quantité de l'AMP cyclique nécessaire à l'activation du domaine régulateur R : la forskoline qui active la biosynthèse de l'AMP cyclique ; la milrinone, supposée agir par son action inhibitrice sur les phosphodiestérases ;
- ceux qui inhibent les protéines phosphatases, enzymes qui régulent la fermeture du canal par déphosphorylation du domaine R, à savoir le bromotétramisol ou certaines xanthines.

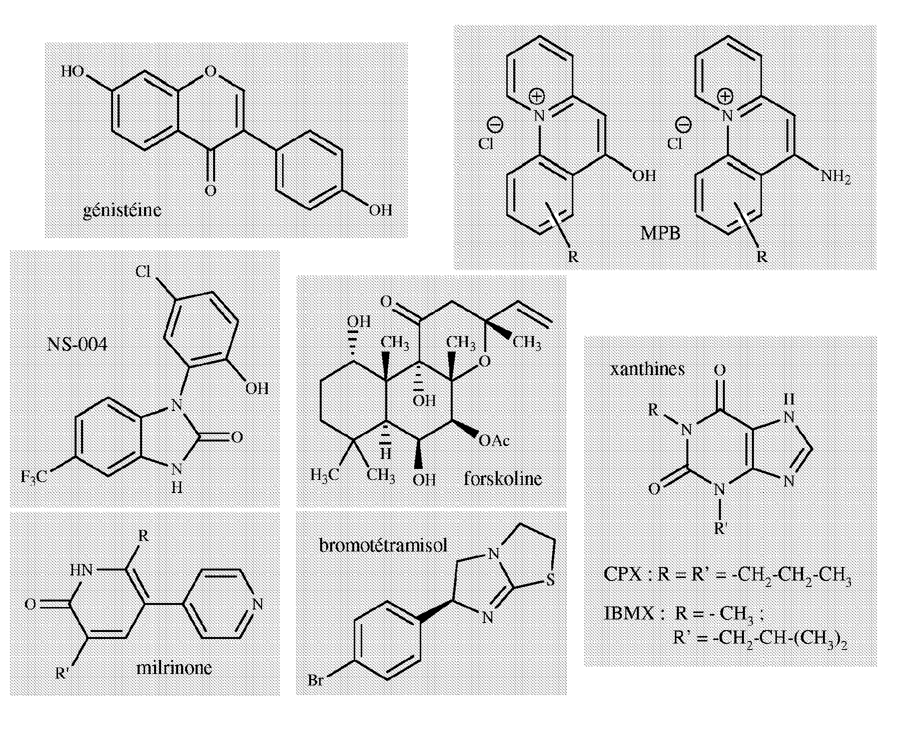

En ce qui concerne les inhibiteurs des canaux CFTR, leur mode d'action consiste généralement au blocage du pore empêchant ainsi les ions de passer à travers le canal [3]. On peut citer, entre autres, des acides carboxyliques, tels que IAA-94 (acide indanyloxyacétique), DPC (diphénylamine-2-carboxylate) et NPPB (5-nitro-2-(3-phénylpropylanzino)benzoate), DIDS (acide 4,4'-diisothiocyanostylbène-2,2'-disulfonique) ou des sulfonylurées dont la glibenclamine fait partie :

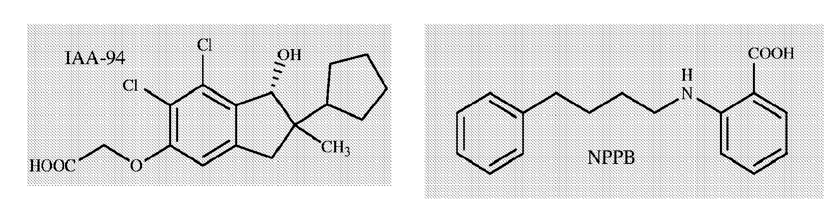

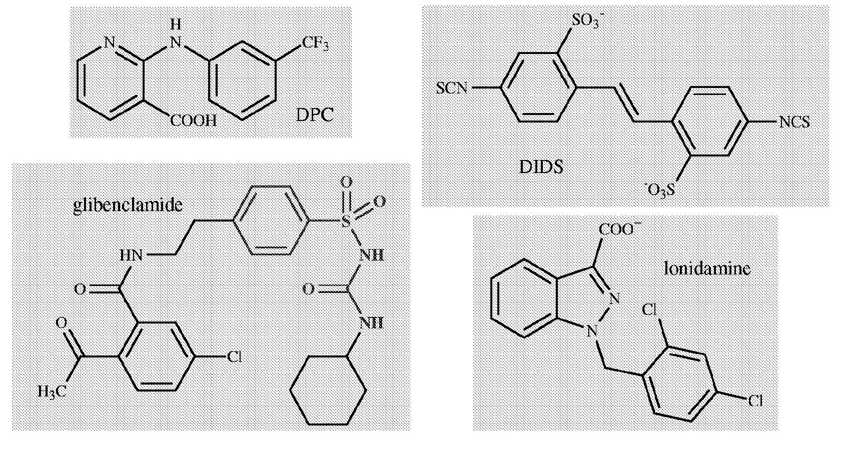

Ces molécules inhibent les canaux CFTR à hautes concentrations et ne sont pas spécifiques de ces canaux. La concentration nécessaire pour inhiber 50% de l'activité du canal CFTR (IC50) par la lonidamine est de 58 µM. Ces inhibiteurs agissent également sur les autres canaux chlorure et les canaux potassiques.

En 2002, Ma *et al*. ont rapporté une nouvelle classe d'inhibiteurs et de nouvelles familles d'activateurs possédant, toutes les deux, des activités très importantes par rapport à celles rapportées jusqu'alors [**5, 6**] :

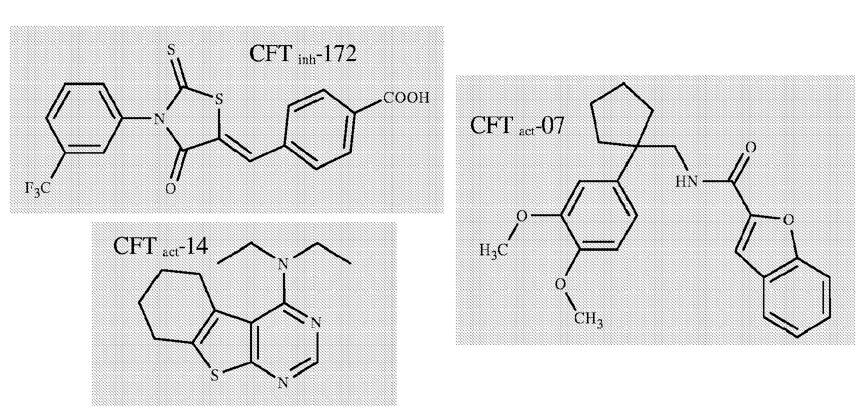

Ces nouveaux inhibiteurs sont de type 2-thiooxo-4-thiazolidinone. Ils possèdent une activité spécifique pour CFTR. L'un d'entre eux, CFTRᵢₙₕ-172, présente une inhibition de 50% (IC50) pour une concentration de 0,2 µM ainsi qu'une absence de toxicité à 7 jours chez la souris pour une injection de 10 mg/kg. Il présente donc les atouts nécessaires à une application thérapeutique. Ainsi, une dose injectée chez la souris (250 µg/kg) réduit les diarrhées provoquées par la toxine du choléra de plus de 90% pendant 6 heures.

En ce qui concerne les nouveaux activateurs, ils ont été mis en évidence par "screening" d'une collection de 60 000 composés. Parmi eux, certains se sont avérés spécifiques de CFTR et particulièrement efficaces avec une concentration nécessaire pour obtenir 50 % de l'activité maximale (EC50) inférieure à 200 nM ainsi qu'une absence de toxicité sur cellules après 24h d'incubation. On peut noter, parmi ces dérivés, la présence de composés hétérocycliques tels que CFTR_{act}-07 et CFTR_{act}-14.

Malgré l'apparition de ces molécules prometteuses et l'arsenal déjà en place, il existe un besoin réel de développer des molécules permettant de moduler le fonctionnement des canaux CFTR et présentant une spécificité, une efficacité et une absence de toxicité, adaptées à des applications thérapeutiques.

Dans le cadre de l'invention, les Demandeurs ont mis en évidence que des molécules, se présentant à pH physiologique sous la forme d'un zwittérion de formule bien déterminée, possédaient une telle activité et de telles propriétés.

Dès lors, l'invention concerne l'utilisation d'une molécule, se présentant à pH physiologique sous la forme d'un zwittérion, de formule : 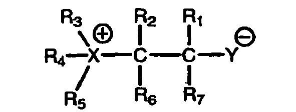 avec X = N ou P ;
Y = O ou S ;
R₁ à R₇ représentent : ⁻ , H, ou des chaînes carbonées, substituées ou non, pouvant contenir des hétéroatomes ;
à l'exception de la bétaïne,
pour la préparation d'un médicament destiné au traitement des maladies associées à un dysfonctionnement des canaux CFTR.

Les molécules concernées par cette application thérapeutique se caractérisent donc par leur activité biologique, en l'occurrence leur capacité à moduler le fonctionnement des canaux CFTR. Par ailleurs, l'exploitation thérapeutique des ces molécules est garantie par leur non toxicité.

On entend par canaux CFTR, à la fois le canal humain isolé mais aussi tous les variants comportant des mutations. Ceci comprend en particulier les protéines des autres espèces animales, mais aussi les protéines naturellement mutées. Une catégorie très importante de canaux CFTR humains mutés concerne les formes rencontrées dans la mucoviscidose, en particulier les mutants ΔF508 (délétion d'une phénylalanine en position 508) et G551D (remplacement d'un résidu glycine par un acide aspartique en position 551).

Ces molécules peuvent avoir un effet inhibiteur ou activateur sur les canaux chlorure CFTR. Leur action est spécifique de ce type de canaux, c'est à dire que ces molécules n'ont en revanche aucun ou peu d'effet sur l'activité des autres canaux à ions (autres canaux à chlorure, canaux potassiques, ...).

Des activateurs auront un effet bénéfique sur les canaux mutés responsables de la mucoviscidose. Dans le cas de canaux non mutés, les molécules activatrices auront des propriétés bronchodilatatrices, bénéfiques dans le cas de nombreuses maladies respiratoires obstructives, telles que l'asthme.

En revanche, les inhibiteurs seront exploités dans le cas de diarrhées, induites par exemple par le bacille responsable du choléra.

La bétaïne est exclue des molécules concernées par l'invention dans la mesure où le document US 5,516,798 divulguait déjà son utilisation pour le traitement des diarrhées. Cependant, le mécanisme d'action n'était absolument pas élucidé dans ce document et aucune formule générale pour les molécules actives ne pouvait en être déduite.

Dans le cadre de l'invention, le terme "pH physiologique" fait référence au pH du corps de l'organisme à traiter, et plus particulièrement au pH à proximité du site d'action de la molécule selon l'invention, à savoir dans l'environnement du canal CFTR ciblé. En pratique, il s'agit de pH compris entre 5 et 9, préférentiellement entre 6 et 8, notamment à pH 7,4.

La molécule selon l'invention se présente sous la forme d'un zwittérion audit pH. Un zwittérion est défini comme une entité qui porte à la fois une charge positive et une charge négative.

Dans les conditions particulières de l'invention, la charge positive est portée par un atome d'azote (N⁺) ou de phosphore (P⁺), alors que la charge négative est liée à la présence d' une fonction carboxylate, hydroxyle ou thiol déprotonée (O⁻ ou S⁻), les deux atomes portant ces charges opposées étant séparés par deux atomes de carbone.

Si la molécule dans l'utilisation revendiquée se trouve sous la forme d'un zwittérion à pH physiologique, elle peut en revanche être préparée et/ou délivrée sous une forme non-zwittérionique, en particulier sous une autre forme acido-basique.

Dans la définition des molécules concernées par la revendication 1, le fait que R₁ à R₇ puissent représenter "-" signifie que l'atome adjacent à R possède au moins une double liaison, comme illustré ci-dessous :

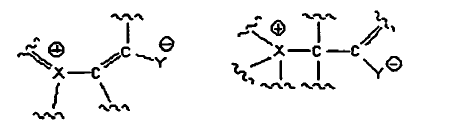

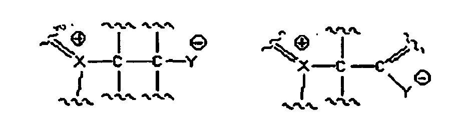

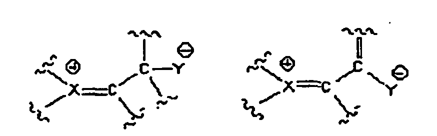

Les Demandeurs ont mis en évidence que la présence d'au moins un cycle dans les molécules définies à la revendication 1 était avantageuse dans le cadre de l'invention. Par conséquent, dans un mode de réalisation préféré, R₁ et/ou R₂ et/ou R₃ et/ou R₄ et/ou R₅ et/ou R₆ et/ou R₇ forment un cycle aromatique ou non aromatique. Il est à noter que cette définition exclut la bétaïne. De nombreux cas de figure sont possibles et en particulier :
- l'atome X est inclus dans un ou deux cycles aromatiques ou non aromatiques ;
- l'atome Y est porté par ou substituant d'un cycle aromatique ou non aromatique ;
- l'atome X est inclus dans un ou deux cycles aromatiques ou non aromatiques et que l'atome Y est porté par un cycle aromatique ou non aromatique ;
- l'atome X est inclus dans un ou deux cycles aromatiques ou non aromatiques et l'atome Y est porté par l'un des cycles aromatiques ou non aromatiques incluant l'atome X.

Dans ce cadre, une première famille de molécules utilisables selon l'invention présente la formule suivante : 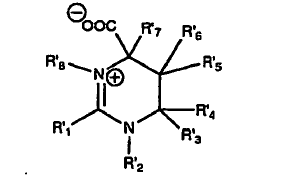 avec R'₁ à R'₈ représentent : ⁻ , H, ou des chaînes carbonées, substituées ou non, pouvant contenir des hétéroatomes ;
R'₁ et R'₂ pouvant former un cycle aromatique ou non aromatique.

Préférentiellement, ces molécules possèdent :
- R'₄=R'₅=OH;
- R'₆ = R'₈ = H;
et avantageusement:
- R'₃ = R'₇ = CH₃.

La charge positive est portée par la fonction imine alors que la charge négative est liée à la présence d'un groupe carboxylate.

Le mode de synthèse de certaines molécules de cette famille est décrit dans Routaboul *et al*. (2002) [7]. Ce document décrit spécifiquement la réaction entre deux molécules de méthylglyoxal et la 2-aminopyridine, ou l'adénine, ou l'adénosine, ou la 2'-désoxyadénosine, ou la 9-propyladénine, ou la cytosine ou l'acide polyadénylique (polyA). Une réaction analogue entre le méthylglyoxal et les résidus arginine des protéines (arginine ou ses dérivés protégés sur le groupe α-amino acide) a déjà été rapportée [**8**]. Ces adduits déjà divulgués sont représentés ci-dessous :

| α-cétoaldéhyde + **X** (dérivés diazotés dont les atomes de N sont séparés par un atome de C, ou amidines au sens large) | **Adduits** (composés de bis addition de l'aldéhyde, ou α-iminoacides cycliques) de **X** : |
|---|---|
| méthylglyoxal + **2-aminopyridine** | 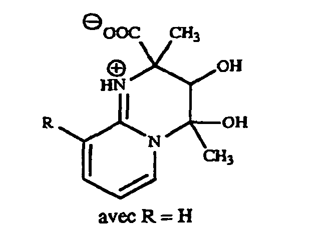 |
| méthylglyoxal + **adénine** | 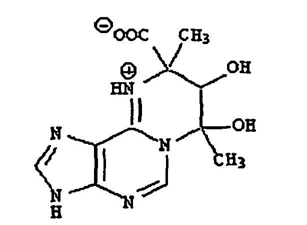 |
| méthylglyoxal + **adénosine** | 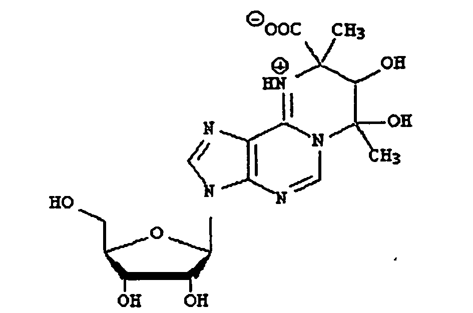 |
| méthylglyoxal + **2'-désoxyadénosine** | 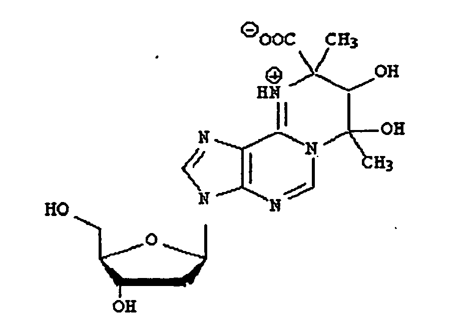 |
| méthylglyoxal + **9-propyladénine** | 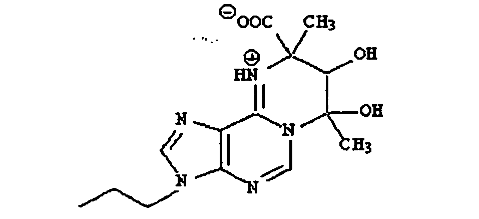 |
| méthylglyoxal + **cytosine** | 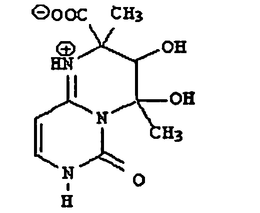 |
| méthylglyoxal + **acide polyadénylique** (polyA) | 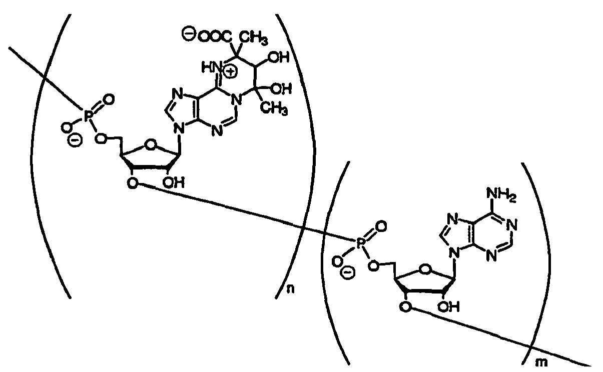 |
| méthylglyoxal + **arginine et ses dérivés** (R) | 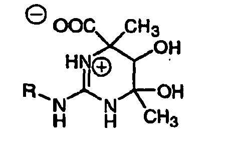 |

En revanche, aucune activité biologique n'avait été rapportée pour ces molécules. Or, les Demandeurs ont constaté que les composés possédant une telle formule étaient des modulateurs de l'activité des canaux CFTR et que par conséquent, ces composés pouvaient être utilisés thérapeutiquement dans le cadre de l'invention.

Par ailleurs, les Demandeurs ont développé de nouvelles molécules sur la base de cette même réaction. Plus précisément, ces nouveaux adduits ont été obtenus par condensation de 2 molécules d'α-cétoaldéhyde possédant des atomes d'hydrogène énolisables sur une molécule possédant deux atomes d'azote séparés par un carbone capables de réagir avec l'α-cétoaldéhyde, en présence d'H₂O et/ou d'un solvant organique tel que l'éthanol, à pH de 1 à 10 et à une température comprise entre 25°C et 80°C. Cette réaction donne naissance à des α-iminoacides, ayant la formule suivante à pH physiologique : 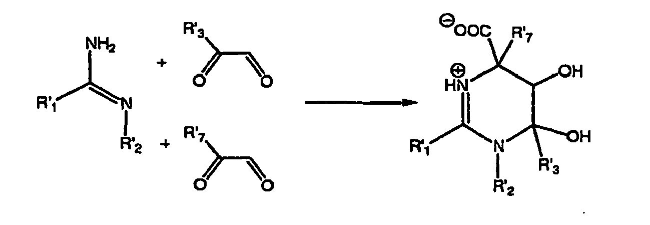 avec R'₁, R'₂, R'₃ et R'₇ représentent H ou des chaînes carbonées, substituées ou non, cycliques ou non, aromatiques ou non, pouvant contenir des hétéroatomes.

L'α-cétoaldéhyde impliqué dans cette réaction est préférentiellement le méthylglyoxal, l'éthylglyoxal, le benzylglyoxal ou un mélange de deux de ces α-cétoaldéhydes. Dans le cas d'un mélange, les 2 α-cétoaldéhydes distincts sont introduits dans le mélange réactionnel d'une manière simultanée ou séquentielle.

La molécule sur laquelle se condensent les 2 molécules d'α-cétoaldéhyde est préférentiellement une base nucléique (adénine ou cytosine) ou ses dérivés (substitués tels que la 9-propyladénine, la 1-propylcytosine ou le polyA), la 2-aminopyridine ou ses dérivés (substitués, 2-amino-3-hydroxypyridine, aminopyrazine ou 1-aminoisoquinoléine), le 2-aminobenzothiazole ou la benzamidine.

Préférentiellement, les nouveaux adduits selon l'invention ont la formule suivante :

| α-cétoaldéhyde + **X** (dérivés diazotés dont les atomes de N sont séparés par un atome de C, ou amidines au sens large) | **Adduits** (composés de bis addition de l'aldéhyde, ou α-iminoacides cycliques) **de X :** |
|---|---|
| méthylglyoxal + **2-amino-3-méthylpyridine** (R=CH3) ou **2-amino-3-hydroxypyridine** (R=OH) | 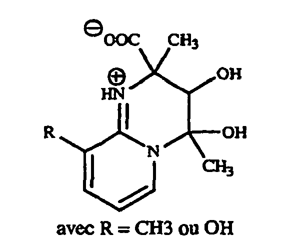 |
| méthylglyoxal + **9-allyladénine** | 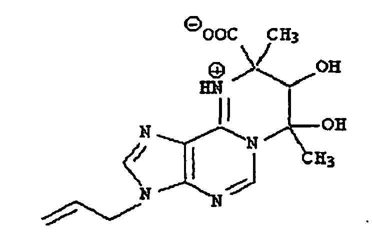 |
| méthylglyoxal + **9-butyladénine** | 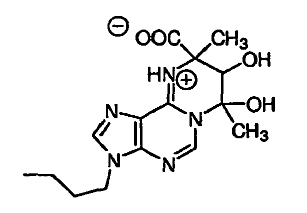 |
| méthylglyoxal + **9-(3-phényl propyl)adénine** | 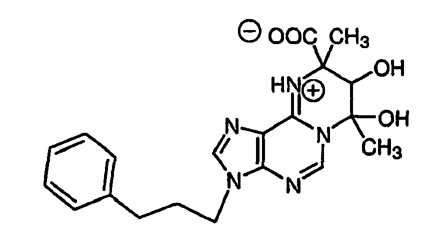 |
| méthylglyoxal + **1-propylcytosine** | 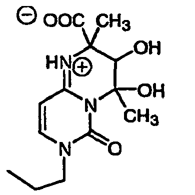 |
| méthylglyoxal + **1-aminoisoquinoléine** | 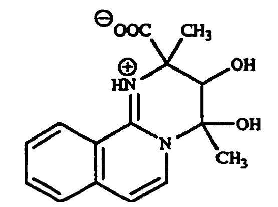 |
| méthylglyoxal + **aminopyrazine** | 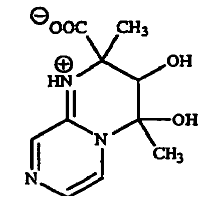 |
| méthylglyoxal + **2-aminobenzothiazole** | 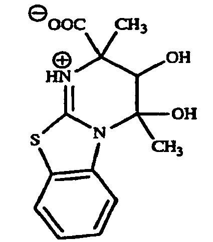 |
| méthylglyoxal + **benzamidine** | 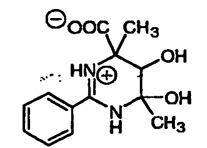 |
| méthylglyoxal + **2-aminoperimidine** | 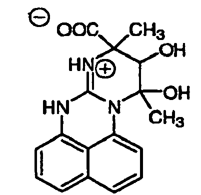 |
| éthylglyoxal + **2-aminopyridine** | 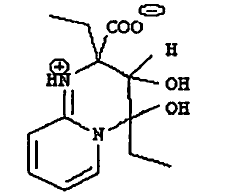 |

Pour l'ensemble de ces molécules, connues ou nouvelles, il est à noter que les fonctions hydroxyles portées par les sucres (par exemple dans le cas des adduits de l'adénosine ou de la 2'-désoxyadénosine) peuvent être modifiées, notamment par acétylation.

A titre d'exemple, les pKa du produit de la réaction entre le méthylglyoxal et la 2-aminopyridine ont été évalués par les Demandeurs, en solution aqueuse à 25°C : il est d'environ 2 pour la fonction acide carboxylique et d'environ 10 pour la fonction imine, ce qui confirme le caractère zwittérionique de la molécule à pH physiologique.

Au cours de cette réaction, on observe la formation de quatre stéréoisomères, séparables sous forme de deux mélanges d'énantiomères, lorsque le dérivé aminé de départ n'est pas chiral. Si il est chiral, quatre diastéréoisomères sont formés et peuvent être isolés sous forme de deux mélanges.

Les Demandeurs ont montré que dans le cas des adduits testés, à savoir ceux de la 9-propyladénine, de l'adénine et de la 1-propylcytosine, l'activité biologique de chacun des diastéréréoisomères séparés (sous la forme de deux mélanges d'énantiomères) comparée à celle de leur mélange était sensiblement la même.

Avantageusement, les molécules utilisées dans le cadre de l'invention pour leur activité vis à vis des canaux CFTR sont les adduits obtenus à partir de 2 molécules de méthylglyoxal et de la 2-amino-3-hydroxypyridine, ou de l'adénine, ou de la 2'-désoxyadénosine, ou de la 9-allyladénine, ou de la 9-propyladénine, ou de la 1-propylcytosine ou de la 1-aminoisoquinoléine. L'adduit entre l'éthylglyoxal et la 2-aminopyridine est également préféré.

Plus généralement, l'invention concerne l'ensemble des adduits, générés par cette réaction, à l'exception de ceux déjà décrits dans les références 7 et 8, ainsi que l'utilisation de l'ensemble de ces molécules comme médicament.

La première utilisation thérapeutique de ces molécules implique leur formulation dans une composition pharmaceutique les intégrant en tant que principe actif.

Dans l'ensemble de la demande, on entend par composition pharmaceutique une composition contenant au moins, à titre de principe(s) actif(s), une molécule selon l'invention en association avec un véhicule physiologiquement acceptable.

Une composition pharmaceutique selon l'invention peut être administrée par voie orale (comprimés ou gélules), par voie parentérale (voie intraveineuse, intramusculaire ou sous-cutanée) ou par voie aérienne.

Des compositions pharmaceutiques selon l'invention se caractérisent en outre en ce qu'elles contiennent des quantités de principe(s) actif(s) adaptées à la posologie, en particulier à la masse des patients et au nombre de prises.

Une seconde série de molécules est obtenue à partir de cette première famille de molécules, par une nouvelle réaction mise en évidence par les Demandeurs.

Ces molécules nouvelles sont des dérivés se présentant sous la forme d'un zwittérion à pH physiologique et de formule générale : 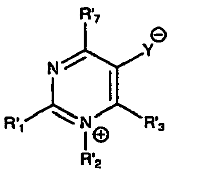 avec R'₁ et R'₂ représentent : ⁻, H, ou des chaînes carbonées, substituées ou non, pouvant contenir des hétéroatomes ; et pouvent former un cycle aromatique ou non aromatique ;
R'₃ et R'₇ représentent : H, ou des chaînes carbonées, substituées ou non, pouvant contenir des hétéroatomes ;
Y = O ou S.

La charge positive est portée par un atome d'azote tétravalent alors que la charge négative est liée à la fonction hydroxyle (fonction phénol) ou thiol déprotonée.

Comme déjà dit, les molécules de la première famille sont obtenues par condensation de 2 molécules d'α-cétoaldéhyde possédant des atomes d'hydrogène énolisables sur une molécule possédant deux atomes d'azote séparés par un carbone capables de réagir avec l'α-cétoaldéhyde, en présence d'H₂O et/ou d'un solvant organique tel que l'éthanol, à pH de 1 à 10 et à une température comprise entre 25°C et 80°C, donnant naissance à un α-iminoacide :

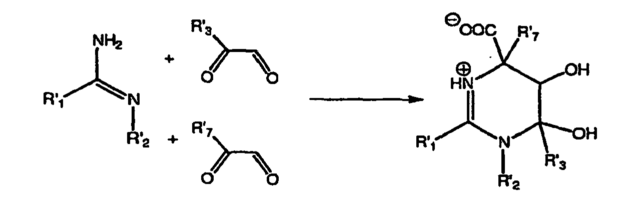

Pour obtenir les molécules de la seconde famille, l'α-iminoacide est chauffé entre 40 et 90°C dans une solution aqueuse de soude ou de potasse 1 à 2M, ce qui aboutit à une décarboxylation oxydante et à une déshydratation selon la réaction suivante :

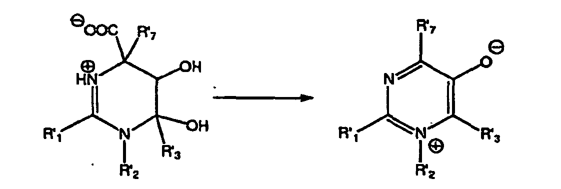

Cette seconde réaction peut également être réalisée en milieu basique organique, par exemple en présence d'éthanolate de sodium dans l'éthanol ou de propanolate de sodium dans le propanol. L'étape d'oxydation peut être spontanée à l'air ou être réalisée à l'aide d'un oxydant tel que la 2,3-dichloro-5,6-dicyanoquinone.

Dans le mode de réalisation où Y=S, l'atome d'oxygène (O) est substitué par un atome de soufre (S).

Dans une étape ultérieure éventuelle, il est possible de condenser de nouvelles molécules, de type aldéhyde, au niveau des résidus R'₃ et/ou R'₇.

Ainsi, les adduits obtenus à partir du méthylglyoxal et de la 2-aminopyridine ont été traités dans ces conditions. Cette réaction a donné lieu à la formation du dérivé suivant :

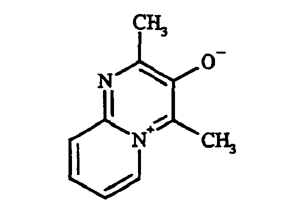

A titre d'exemple, le pKₐ de ce composé a été évalué à 4. Cela signifie qu'en solution aqueuse à pH 7, ce composé est principalement sous forme zwittérionique, ainsi que dans le cas de solutions très diluées, telles que celles utilisées pour les tests biologiques.

Cette réaction, bien que non complète, a également eu lieu avec, par exemple :
- les adduits de la 1-aminoisoquinoléine : 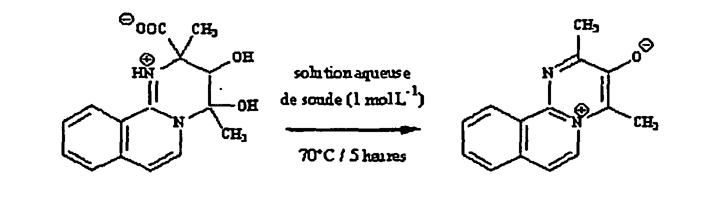
- les adduits du 2-aminobenzothiazole : 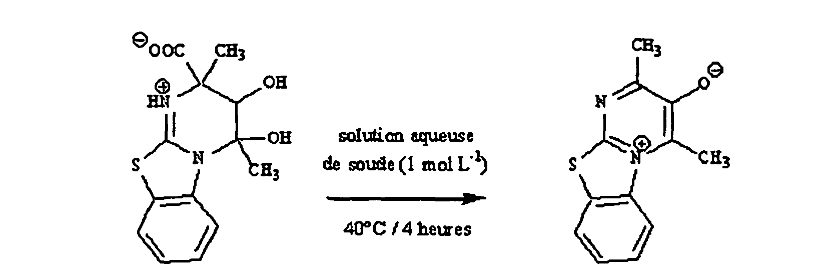
- les adduits de la benzamidine :

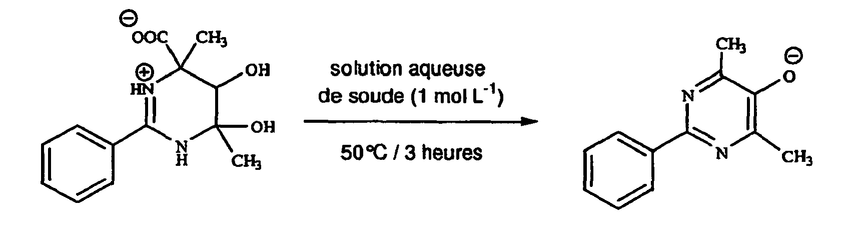

A partir de ces composés, il est possible d'obtenir une nouvelle série de molécules :
- par substitution de l'atome d'oxygène (O) par un atome de soufre (S), donnant naissance à un composé soufré. Cette réaction est exemplifiée ci-dessous: 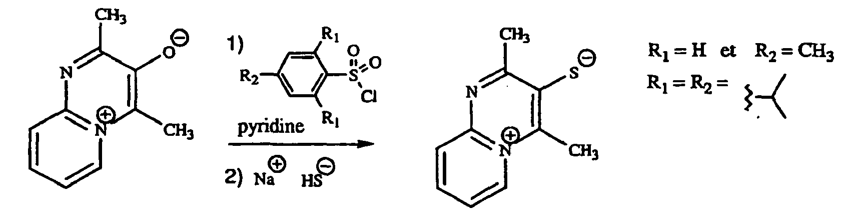
   Cette transformation s'effectue en deux étapes: une sulfonylation sur l'atome d'oxygène puis la substitution du groupement arylsulfonyle par réaction avec un ion hydrogénosulfure.
- et/ou par condensation au niveau des groupements -CH3 situés de part et d'autre du groupement hydroxyle , par exemple avec le formaldéhyde :

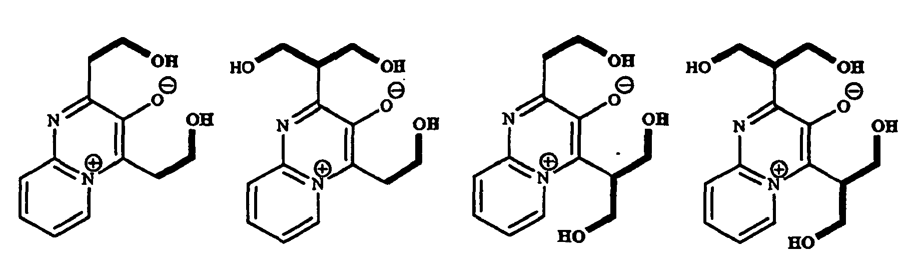

Les atomes d'hydrogènes des groupements méthyles sont en effet acides et peuvent être substitués par réaction avec des électrophiles en milieu basique.

Ainsi, une réaction équivalente peut être réalisée avec, par exemple, le benzaldéhyde :

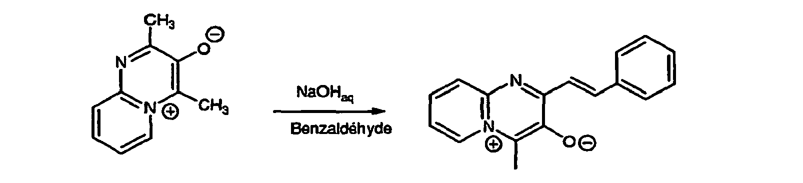

La réaction (condensation et déshydratation) est menée dans l'obscurité en dissolvant le dérivé oxygéné dans une solution aqueuse de soude 2M, puis en ajoutant le benzaldéhyde (ou un dérivé) en léger excès. Le dérivé recherché pur est obtenu sous la forme d'une poudre rouge après chromatographie sur colonne de silice en éluant avec un mélange dichlorométhane-méthanol. La réaction peut être réalisée en présence d'un large excès de benzaldéhyde ou d'un dérivé, de manière à faire réagir de façon similaire le second groupe méthyle et d'obtenir ainsi le dérivé disubstitué.

Une molécule de formule suivante a ainsi été obtenue :

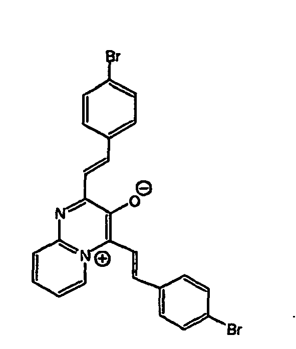

Outre le fait d'être nouvelles, les Demandeurs ont montré que les molécules se présentant sous la forme d'un zwittérion à pH physiologique, de formule générale : 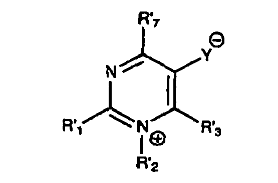 avec R'₁, R'₂, R'₃ et R'₇ représentent: ⁻ , H, ou des chaînes carbonées, substituées ou non, pouvant contenir des hétéroatomes ;
R'₁ et R'₂ pouvant former un cycle aromatique ou non aromatique ;
Y = O ou S,
étaient susceptibles d'avoir des applications thérapeutiques, en particulier dans la préparation de médicaments pour le traitement des maladies associées à un dysfonctionnement des canaux CFTR.

Avantageusement, dans cette dernière application thérapeutique, les molécules de cette deuxième famille ont une formule choisie dans le groupe comprenant :

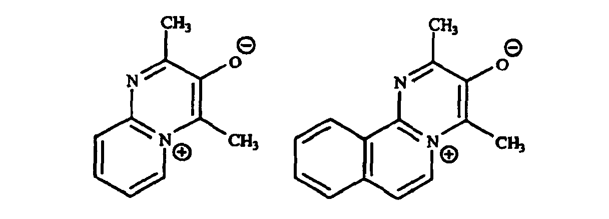

Enfin, il existe un certain nombre de molécules déjà connues en tant que telles et se présentant sous la forme d'un zwittérion de la formule revendiquée à pH physiologique, susceptible de posséder l'activité biologique selon l'invention.

Une molécule répondant à cette définition est la 7-méthylguanosine, décrit entre autre dans le document Ermolinsky *et al.* [9] :

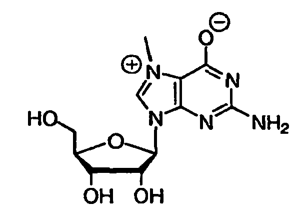

Là encore, les fonctions hydroxyles portés par les sucres peuvent être modifiées, notamment par acétylation.

A la connaissance des Demandeurs, les molécules répondant à une telle définition n'ont jamais été décrites dans le cadre en relation avec des applications thérapeutiques. De telles molécules pour leur utilisation comme médicament, en particulier destiné au traitement de maladies associées à un dysfonctionnement des canaux CFTR font donc également partie de l'invention.

Les avantages qui découlent de la présente invention sont illustrés dans les exemples de réalisation présentés ci-dessous, à l'appui des figures annexées.

**Figure 1** **:** Effet des composés de l'invention (composé 1 à 4, 6 à 7 et 9 à 10), à une concentration de 1 µM, sur le récepteur CFTR surexprimé dans les cellules CHO-WT. La référence 100% d'activation correspond à l'activation observée en présence de 1 µM de forskoline.

**Figure 2** **:** Courbes de détermination :
A) des EC50 pour les composés activateurs (composés 9 et 10), établies en présence de 1 µM de forskoline ;
B) des IC50 pour les composés inhibiteurs (composés 1 à 4 et 6 à 7), établies en présence de 5 µM de forskoline.

### I/ MATERIEL ET METHODES

### 1/ Synthèse des composés de la famille I (composés 1 à 5 et 9 à 10) :

a- *Procédure générale* : Le principe de cette synthèse a été décrit pour l'essentiel dans Routaboul *et al*. (2002) [7]. Une 2-aminopyridine est dissoute dans la solution aqueuse commerciale de méthylglyoxal à 40%. Le mélange est chauffé à 50°C sous argon. Après évaporation sous pression réduite, le mélange des deux isomères est séparé du milieu réactionnel par chromatographie sur cartouche de phase inverse C₁₈ en éluant avec de l'eau. Après évaporation du solvant, le mélange de diastéréoisomères est dissout dans un minimum de méthanol et précipité dans l'éther anhydre. Pour certains adduits, les diastéréoisomères sont séparés par chromatographie sur colonne de gel de silice (dépôt sec ; élution : gradient CH₂Cl₂ / MeOH [9:1] à [7 : 3]). Après évaporation du solvant sous pression réduite, ils sont dissous de l'eau et purifiés par une nouvelle chromatographie sur cartouche de phase inverse C₁₈ (éluant : eau). Les composés se présentent sous forme de poudres blanches.
*b- Composé 1* : Adduits de la 2'-désoxyadénosine 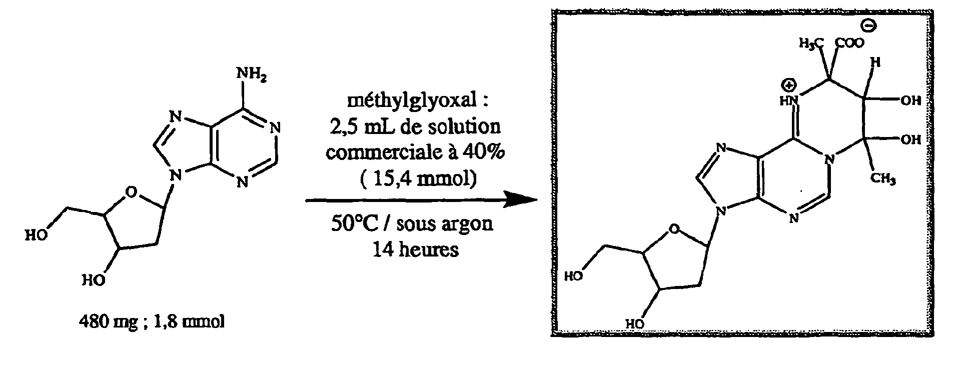
   Le rendement de la réaction a été de 20% pour l'adduit majoritaire (150 mg ou 0,35 mmol) et de 7% pour l'adduit minoritaire (53 mg ou 0,12 mmol).
*c- Composé 2* : Adduits de la 1-propylcytosine 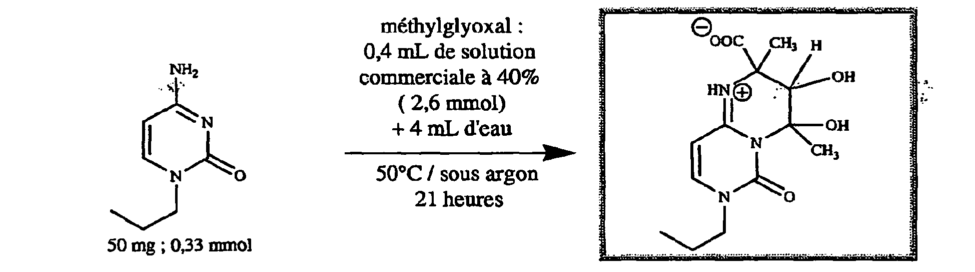
   Un mélange des adduits diastéréoisomères a été obtenu avec un rendement de 74% (73 mg ou 0,25 mmol).
*d- Composé 3* : Adduits de la 2-amino-3-hydroxypyridine 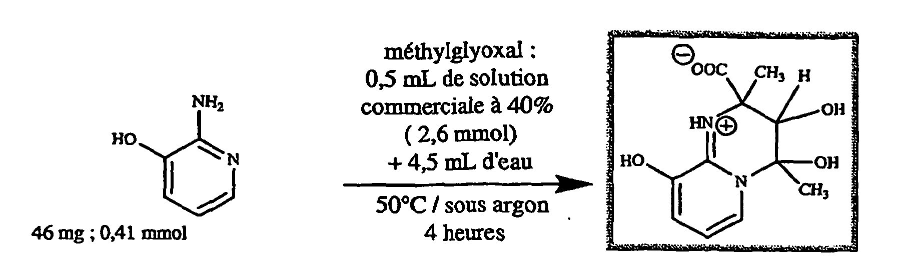
   Un mélange des adduits diastéréoisomères a été obtenu avec un rendement de 73% (77 mg ou 0,30 mmol).
*e- Composé 4* : Adduits de la 1-aminoisoquinoléine 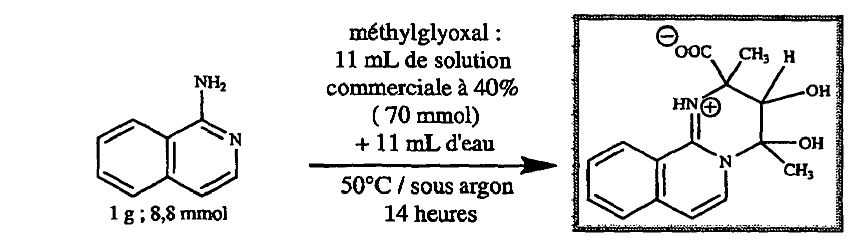
   Le mélange des adduits diastéréoisomères précipite et est séparé du milieu réactionnel par filtration et lavage avec un minimum d'éther diéthylique anhydre. Il est obtenu avec un rendement de 82% (2,065 g ou 7,2 mmol).
*f- Composé 5* : Adduits de l'adénine 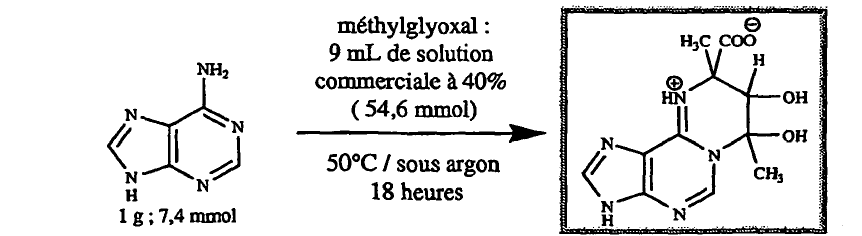
   Le rendement de la réaction a été de 46% pour l'adduit majoritaire (0,950 g ou 3,4 mmol) et de 20% pour l'adduit minoritaire (0,413 g ou 1,48 mmol).
*g- Composé 9* : Adduits de la 9-propyladénine 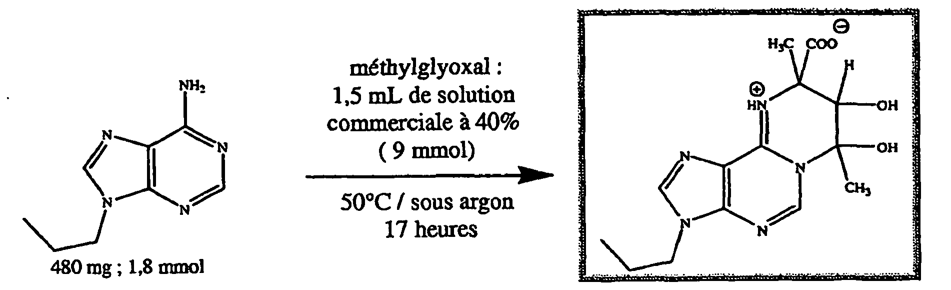
   Le rendement de la réaction a été de 24% pour l'adduit majoritaire (109 mg ou 0,34 mmol) et de 17% pour l'adduit minoritaire (76 mg ou 0,24 mmol).
*h- Composé 10* : Adduits de la 9-allyladénine

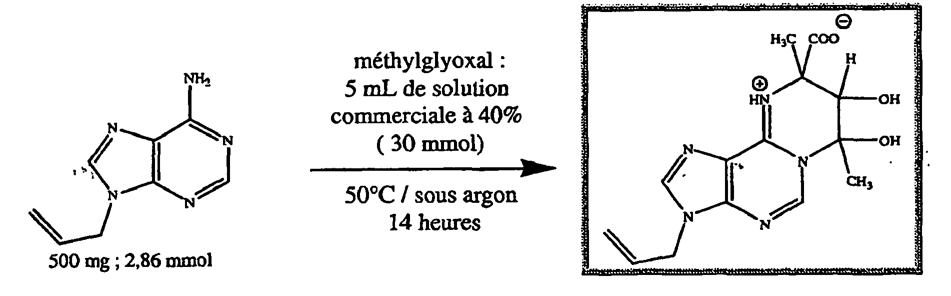

Après séparation du milieu réactionnel sur cartouche de phase inverse C₁₈, l'adduit majoritaire est isolé et cristallisé dans le propan-2-ol. On en obtient 390 mg ou 1,22 mmol, soit un rendement de 43%.

### 2/ Synthèse des composés de la famille II (composés 6 et 7) :

a- *Procédure générale* : Les composés de la famille II sont obtenus à partir des composés de la famille I, par chauffage en milieu basique.
*b- Composé 6* : Dérivé de la 2-aminopyridine
   1^{ère} étape : Synthèse des adduits de la 2-aminopyridine 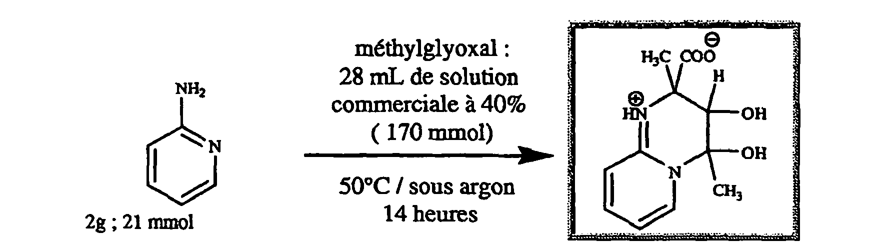 2^{ème} étape : Synthèse du dérivé de la 2-aminopyridine 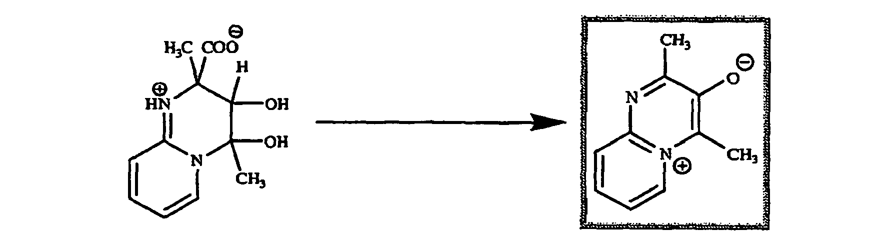
   Le mélange des diastéréoisomères (200 mg, 0,84 mmol) en solution dans de la soude (1 mol L⁻¹, 25 mL) est chauffé à 70°C pendant 4,5 heures. Après refroidissement, la solution est neutralisée (pH 7,2) avec une solution d'acide chlorhydrique. Le produit est dessalé par filtration sur cartouche de phase inverse C₁₈ (éluant eau puis méthanol) puis purifié par une nouvelle chromatographie sur cartouche de phase inverse. Après évaporation du solvant, le produit est obtenu sous forme d'un solide jaune (119 mg ; 0,68 mmol), avec un rendement de 81%.
*c*- *Composé 7* : Dérivé de la 1-aminoisoquinoléine 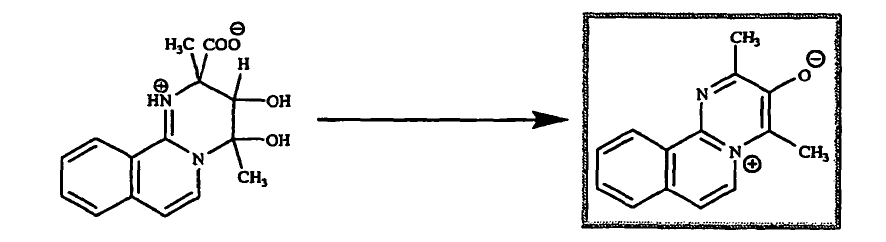
   Le mélange des diastéréoisomères (150 mg, 0,52 mmol), décrit à la section 1-e (composé 4), en solution dans de la soude (1 mol L⁻¹, 2 mL) est chauffé à 70°C pendant 5 heures. Après refroidissement, la solution est neutralisée (pH 7,2) avec une solution d'acide phosphorique. Après extraction au dichlorométhane (3 fois 10 mL) et évaporation du solvant, le résidu est chromatographié sur colonne de silice (dépôt : dichlorométhane, élution : mélange dichlorométhane / méthanol). Après évaporation du solvant, deux produits sont isolés : le dérivé attendu sous forme d'un solide jaune (8 mg, 0,04 mmol, 8 %) ainsi que la 1-aminoisoquinoléine (21 mg, 0,15 mmol, 28%) caractérisée par spectroscopie de RMN ¹H et ¹³C.

3/ Obtention du composé 8 : 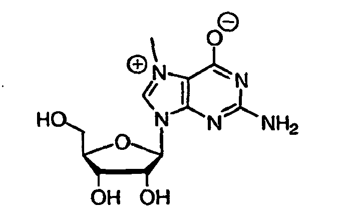
   Les informations nécessaires à l'obtention de la 7-méthylguanosine sont disponibles dans le document Ermolinsky *et al.* [**9**].
4/ Cultures cellulaires : Des cellules CHO (lignée cellulaire Chinese Hamster Ovary) ont été transfectées de manière stable par un vecteur contenant le gène codant le récepteur CFTR sauvage (CHO-WT) ou un gène muté codant un récepteur dont le résidu glycine en position 551 est substitué par un acide aspartique (CHO-G551D). Les cellules ont été cultivées à 37°C en présence de 5% de CO2 et maintenues dans du milieu MEM contenant 7% de sérum foetal bovin, 0.5% d'antibiotiques (50UI/ml de pénicilline et 50µg/ml de streptomycine), ainsi que 100 µM ou 20 µM de méthotréxate.
   Les expériences ont également été réalisées sur des cellules Calu-3, une lignée cellulaire humaine pulmonaire qui exprime naturellement la protéine CFTR sauvage, cultivées comme décrit précédemment et sur des cellules CF15 qui sont des cellules épithéliales humaines d'origine nasale exprimant le gène muté F508-CFTR (délétion d'un résidu phénylalanine en position 508).
5/ Activité biologique des canaux CFTR : L'activité des canaux à ions chlorure a été évaluée grâce à la mesure de l'efflux d'iodure radioactif ¹²⁵I sortant des cellules CHO transfectées. Toutes les expériences ont été réalisées par un système robotique (Perkin Elmer Life Sciences, Courtaboeuf, France). Les cellules ont été cultivées dans des plaques à 24 puits afin de réaliser des expériences parallèles et d'effectuer une étude comparative. Au début de chaque expérience, les cellules ont été lavées avec du tampon d'efflux contenant (en mM): 137 NaCl, 5.36 KCl, 0.8 MgCl₂, 5.5 glucose et 10 HEPES, pH 7.4. Les cellules ont ensuite été incubées dans du tampon d'efflux contenant 1 µM KI et 1 µCi Na¹²⁵I/ml (NEN, Boston, MA) pendant 30 min à 37°C pour permettre au ¹²⁵I d'atteindre l'équilibre. Les cellules ont ensuite été lavées avec du milieu d'efflux pour enlever le ¹²⁵I extracellulaire. La perte de ¹²⁵I intracellulaire a été déterminée en prélevant le milieu avec du tampon d'efflux chaque minute, pendant 11 min. Les 4 premiers aliquotes ont été utilisés pour établir une ligne basale stable dans le tampon d'efflux seuL La radioactivité résiduelle a été extraite à l'aide de NaOH 0.1N, et déterminée grâce à un compteur gamma Packard Cobra II (Perkin Elmer Life Sciences, Courtaboeuf, France). Les résultats représentent la moyenne ± la déviation standard (SD) de 4 expériences indépendantes.
6/ Test de cytotoxicité : Le test de toxicité au MTT est un test colorimétrique qui repose sur la capacité des déshydrogénases mitochondriales à métaboliser le MTT (sel de tetrazolium jaune) en formazan (pourpre). L'absorbance, proportionnelle à la concentration de colorant converti, peut être alors mesurée par spectrophotométrie. Les cellules CHO sont mises à incubées sur des plaques 96 puits en présence de l'agent à tester pendant 2 h. 3 contrôles sont réalisés: 100% cellules vivantes: cellules sans agent; 0% cellules vivantes: cellules laissées à l'air libre ; blanc : milieu sans cellule. Les cellules sont rincées avec du milieu RPMI sans rouge de phénol pour que la couleur du milieu n'interfère pas dans les mesures de l'absorbance. Puis, elles sont incubées pendant 4 h avec 100µl de solution de RPMI supplémentée en MTT (0,5mg/ml). Le milieu est alors éliminé, l'ajout de 100 µl de DMSO permet de solubiliser le colorant converti (formazan). L'absorbance est mesurée par spectrophotométrie à570 nm (pourpre) et à 630 nm (bruit de fond). Afin de s'affranchir du bruit de fond, le calcul suivant est effectué: DO_{réelle}=DO₅₇₀ₙₘ-DO₆₃₀ₙₘ. Puis, les résultats sont normalisés par rapport aux contrôles (100% et 0% de cellules vivantes) et sont présentés sous forme de moyenne +/- SEM.
7/ Ajout d'agents pharmacologiques : les molécules de l'invention (composés 1 à 10) ainsi que des activateurs déjà connus des canaux CFTR (forskoline et génistéine) ont été ajoutés aux concentrations indiquées.

### II/ RESULTATS

### 1/ Effet des composés testés sur l'activité des récepteurs CFTR :

L'effet des différents composés synthétisés sur l'activité des canaux à chlorure CFTR a été testé sur des cellules CHO surexprimant le récepteur sauvage (CHO-WT). Un niveau d'activation 100% de référence a été choisi pour une activation en présence de 1 µM de forskoline (Fsk). Les composés testés ont été ajoutés à une concentration de 1 µM. Dans le cas d'une augmentation significative, le composé a été répertorié comme un activateur. Dans le cas d'une activation significativement inférieure à 100% en présence du composé, celui-ci a été répertorié comme un inhibiteur.

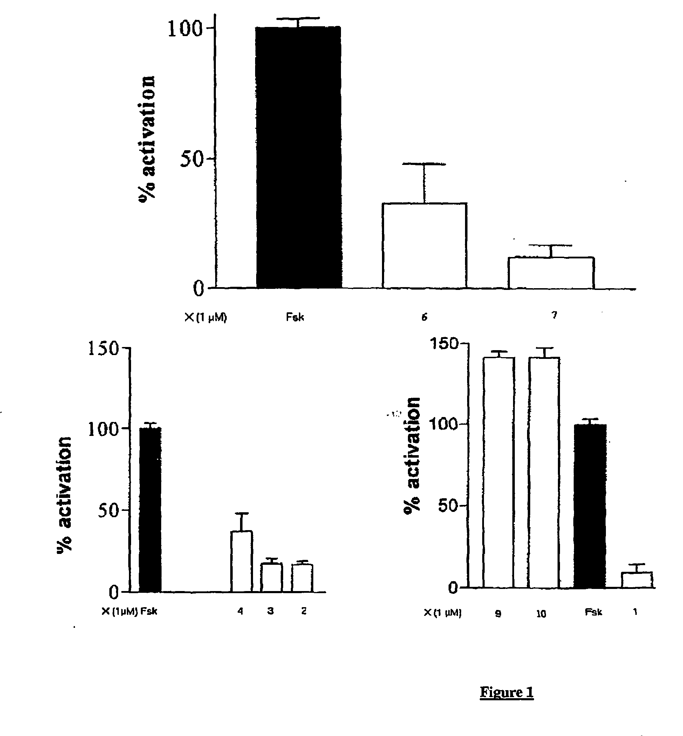

Des exemples de diagramme obtenus sont illustrés à la figure 1.

Afin d'affiner cette étude, les valeurs de IC50 pour les inhibiteurs et de EC50 pour les activateurs ont été déterminées. Le pourcentage d'activation est suivi en présence de concentrations croissantes du composé testé. Dans le cas des inhibiteurs, l'activation 100% a été obtenue en présence de forskoline 5 µM. Dans le cas des activateurs, le pourcentage d'activation a été suivie en présence de forskoline 1 µM.

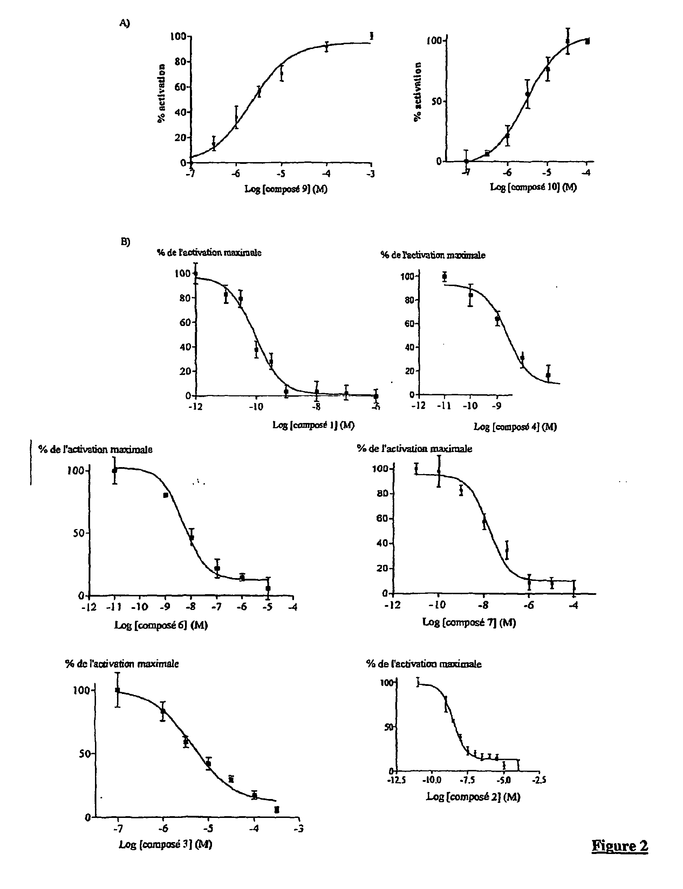

Des exemples de courbes effet/dose permettant de déterminer les valeurs EC50 et IC50 sont illustrés à la figure 2.

Une étude a été mené pour les 11 composés synthétisés sur 4 lignées cellulaires différentes (CHO-wt, CHO-G551D, Calu3, CF15). Les inhibiteurs glibenclamide et bétaïne ont également été incorporés à cette étude. Il est à noter que pour les effets sur les cellules CF15, le flux d'ions iodure a été mesuré après 24 h d'incubation à 27°C. Toutes les mesures ont été faites en présence de Fsk 5 µm (CHO-wt et Calu-3) ou 10 µm Fsk + 30 µm génistéïne (G551D-CFTR et CF15). Les dérivés testés ont été ajoutés directement en solution alors que le témoin glibenclamide a été incubé 30 minutes avant la mesure du flux d'ions iodure. Les résultats sont présentés dans le tableau I ci-dessous :

| Composé | Formule | IC50 ou EC50 | | | | |
|---|---|---|---|---|---|---|
| | | Effet | cellules CHO-WT | CHO-G551D | cellules pulmonaires humaines Calu3 | cellules CF15 |
| Glibenclamide | Voir page 4 | - | 15 µM | 8 µM | 12 µM | 12 µM |
| Bétaïne | | - | 96 µM | | | |
| 1 | 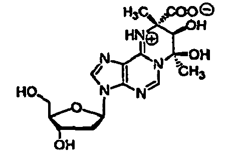 | - | 71 pM | 43 nM | 93 pM | 68 pM |
| 2 (mélange d'isomères 2a et 2b) | 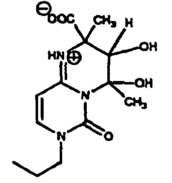 | - | 2 nM | 154 nM | 2 nM | 6 nM |
| 2a (isomère majoritaire) | 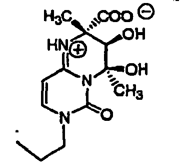 | - | 2 nM | | | |
| 2b (isomère minoritaire) | 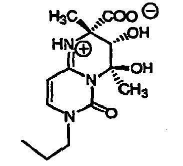 | - | 1,5 nM | | | |
| 3 (mélange d'isomères) | 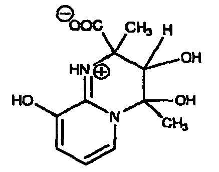 | - | 4 µM | 36 µM | 11 µM | 7 µM |
| 4 (mélange d'isomères) | 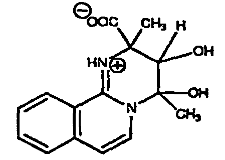 | - | 2,5 nM | 190 nM | 16 nM | 9 nM |
| 4a (isomère majoritaire) | 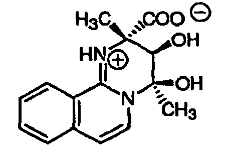 | - | 3nM | | | |
| 5 (mélange d'isomères 5a et 5 b) | 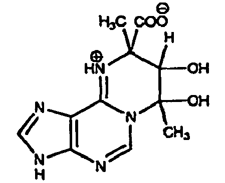 | - | 6 µM | 110 µM | 12 µM | 6 µM |
| 5a (isomère majoritaire) | 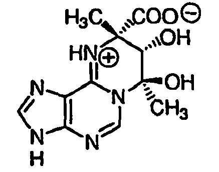 | - | 10 µM | | | |
| 5b (isomère minoritaire) | 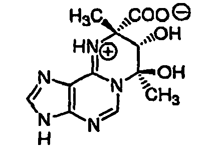 | - | 3 µM | | | |
| 6 | 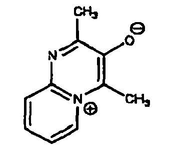 | - | 5 nM | 90 nM | 5 nM | 10 nM |
| 6 (chlorhydrate) | 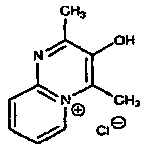 | - | 7nM | | | |
| 7 | 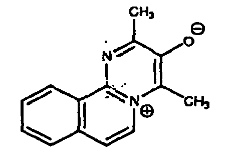 | - | 16nM | 60nM | 17 nM | 11 nM |
| 8 | 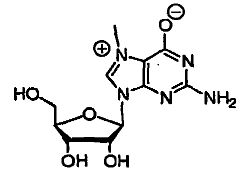 | - | 3,5 µM | 15 µM | 11 µM | 11 µM |
| 9 (mélange d'isomères) | 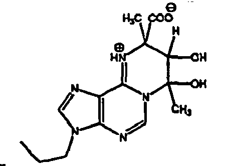 | + | 2 µM | | | |
| 9a (isomère majoritaire) | 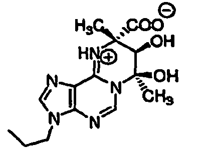 | + | 2 µM | | 2 µM | |
| 9b (isomère minoritaire) | 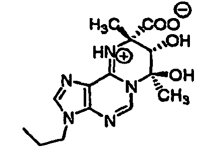 | + | 3 µM | | | |
| 10 (mélange d'isomères) | 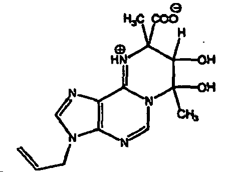 | + | 7,5 µM | | | |
| 11 (mélange d'isomères) | 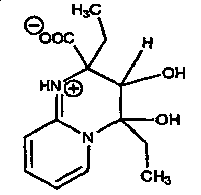 | - | 18 µM | | | |

Les résultats montrent que les effets observés, tant d'un point de vue qualitatif que quantitatif, sont conservés :
- entre des cellules exprimant le récepteur sauvage (Calu3) et des cellules surexprimant cette même protéine dans une lignée cellulaire animale (CHO-WT),
- pour le composé 9 de la première famille, entre les adduits majoritaire et minoritaire séparés et le mélange des 2 diastéréosiomères.

Par ailleurs, on constate que la plupart des molécules présentent une activité inhibitrice de la protéine CFTR Il est à noter que l'effet inhibiteur mesuré est généralement bien meilleur que celui des inhibiteurs jusqu'alors décrits dans la littérature. De même, les activateurs ont un effet significatif et pourront être exploités pour les effets bronchodilatateurs induits.

Enfin, des tests parallèles menés sur d'autres canaux ioniques ont révélé que ces composés ne présentaient aucune ou peu d'activité envers ces canaux (résultats non montrés). Leur action sur les canaux CFTR est donc spécifique et ciblée, offrant à ce titre des potentiels thérapeutiques.

### 2/ Effet des composés testés sur la viabilité cellulaire :

La toxicité des composés présentant l'activité biologique d'intérêt a été testée. Pour cela, des cellules CHO ont été incubées avec lesdits composés pendant une durée de 2 à 24 heures et à une concentration de 10 à 100 µM. Leur toxicité a été estimée par mesure de la viabilité cellulaire, comparée à celle de cellules sans agent (100% de cellules vivantes) celle de cellules laissées à l'air libre (0% de cellules vivantes).

Les résultats obtenus pour les composés 1 à 4, 6 à 7 et 9 à 10 de l'invention (non montrés) ont révélé qu'ils ne présentaient aucune cytotoxicité notable, quelles que soient la durée et la concentration d'incubation utilisées. Leur application thérapeutique est donc tout à fait envisageable.

### 3/ Effet des composés testés sur des récepteurs mutés ou en présence de différents composés pharmacologiques :

Dans une étude plus détaillée, les Demandeurs se sont intéressés à l'effet d'un inhibiteur (composé 1) ou d'un activateur (composé 9), selon l'invention, sur le récepteur CFTR :
- sur un récepteur sauvage (CHO-WT) ou sur un récepteur muté (CHO-G551D), rencontré dans certaines formes de mucoviscidose ;
- en présence d'activateurs déjà connus, tels que la forskoline qui augmente la disponibilité en AMPc impliquée dans l'activation du domaine régulateur R, ou la génistéine qui agit directement sur les domaines du fixation des nucléotides et prolonge l'ouverture des canaux.

Les résultats obtenus sont présentés dans le Tableau II :

**Tableau II : Effets du composé 1 et du composé 9 sur l'activation de CFTR sauvage (CHO-WT) ou muté (CHO-G551D), en présence de divers activateurs.**

| **Activateur** | Composé(s) ajouté(s) | CHO-WT | CHO-G551D |
|---|---|---|---|
| **1 µM Fsk** | (activateur) | activation | pas d'effet |
| **1 µM Fsk** | + 1 µM composé 1 (inhibiteur) | inhibition | pas d'effet |
| **5 µM Fsk** | + 1 µM composé 1 (inhibiteur) | inhibition | pas d'effet |
| **1 µM Fsk** | + 1 µM composé 9 | activation | pas d'effet |
| | (activateur) | (potentialisation) | |
| **10 µM fsk+ 30 µM génistéine** | | activation | activation |
| **10 µM fsk+ 30 µM génistéine** | | inhibition | inhibition |
| + 1 µM composé 1 (inhibiteur) | | | |
| **10 µM fsk+ 30 µM génistéine** | | activation | activation |
| + 1µM composé 9 (activateur) | | | |

Ces expériences révèlent que:
- le composé 1 antagonise efficacement l'action des activateurs forskoline et génistéine, et cela sur les récepteurs sauvages comme sur les récepteurs mutés ;
- le composé 9 a un effet activateur sur le récepteur sauvage mais est incapable d'activer le récepteur muté G551D. En revanche, il est capable d'activer une autre forme mutée du récepteur, ΔF508 (résultats non montrés).

### BIBLIOGRAPHIE

1. Al-Awqati, Q. (2002). Alternative treatment for secretory diarrhea revealed in a new class of CFTR inhibitors. J Clin Invest 110, 1599-1601.
2. Akabas, M. H. (2000). Cystic fibrosis transmembrane conductance regulator. Structure and function of an epithelial chloride channel. J Biol Chem 275, 3729-3732.
3. Hume, J. R., Duan, D., Collier, M. L., Yamazaki, J., et Horowitz, B. (2000). Anion transport in heart. Physiol Rev 80, 31-81.
4. Sheppard, D. N., et Welsh, M. J. (1999). Structure and function of the CFTR chloride channel. Physiol Rev 79, S23-45.
5. Ma, T., Thiagarajah, J. R., Yang, H., Sonawane, N. D., Folli, C., Galietta, L. J., et Verkman, A. S. (2002a). Thiazolidinone CFTR inhibitor identified by high-throughput screening blocks cholera toxin-induced intestinal fluid secretion. J Clin Invest 110, 1651-1658.
6. Ma, T., Vetrivel, L, Yang, H., Pedemonte, N., Zegarra-Moran, O., Galietta, L. J., et Verkman, A. S. (2002b). High-affinity activators of cystic fibrosis transmembrane conductance regulator (CFTR) chloride conductance identified by high-throughput screening. J Biol Chem 277, 37235-37241.
7. Routaboul , C., Dumas, L., Gautier-Luneau, L, Vergne, J., Maurel, M. C., et Décout, J. L. (2002). New stereoselective reaction of methylglyoxal with 2-aminopyridine and adenine derivatives: Formation of imino acid-nucleic base derivatives in water under mild conditions. Chem Commun, 1114-1115.
8. Oya, T., Hattori, N., Mizuno, Y., Miyata, S., Maeda, S., Osawa, T., et Uchida, K. (1999). Methylglyoxal modification of protein. Chemical and immunochemical characterization of methylglyoxal-arginine adducts. J Biol Chem 274, 18492-18502.
9. Ermolinsky, B., Efimtseva, E., Alexeev, C., Mikhailov, S., Balzarini, J., et De Clercq, E. (2003). Dinucleoside Monophosphates containing AZT and 1-methyladenosine or 7-methylguanosine. Nucleosides, Nucleotides and Nucleic Acids 22(5-8), 853-855.

## Revendications

1. Utilisation d'une molécule, se présentant à pH physiologique sous la forme d'un zwittérion, de formule : 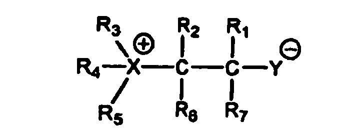 avec X = N ou P ;
Y = O ou S ;
R₁ à R₇ représentent : ⁻ , H, ou des chaînes carbonées, substituées ou non, pouvant contenir des hétéroatomes ;
à l'exception de la bétaïne,
pour la préparation d'un médicament destiné au traitement des maladies choisies dans le groupe comprenant : la mucoviscidose, les maladies bronchostrictives, telles que l'asthme, ou la diarrhée.

2. Utilisation selon la revendication 1, ***caractérisée* en ce que** R₁ et/ou R₂ et/ou R₃ et/ou R₄ et/ou R₅ et/ou R₆ et/ou R₇ forment un cycle aromatique ou non aromatique.

3. Utilisation selon la revendication 2, ***caractérisée* en ce que** la molécule présente la formule suivante : 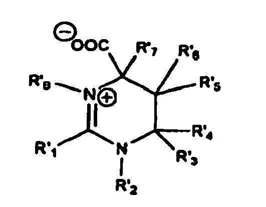 avec R'₁ à R'₈ représentent : ⁻, H, ou des chaînes carbonées, substituées ou non, pouvant contenir des hétéroatomes ;
R'₁ et R'₂ pouvant former un cycle aromatique ou non aromatique.

4. Utilisation selon la revendication 3, ***caractérisée* en ce que** :
- R'₄ = R'₅ = OH ;
- R'₆ = R'₈ = H.

5. Utilisation selon la revendication 4, ***caractérisée* en ce que** :
- R'₃ = R'₇ = CH₃.

6. Utilisation selon la revendication 5, ***caractérisée* en ce que** la molécule a une formule choisie dans le groupe comprenant :

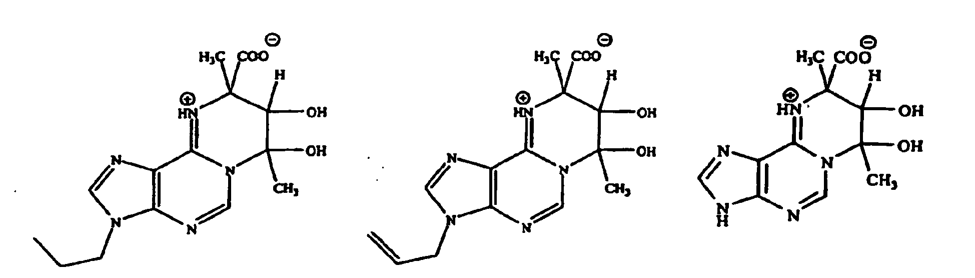

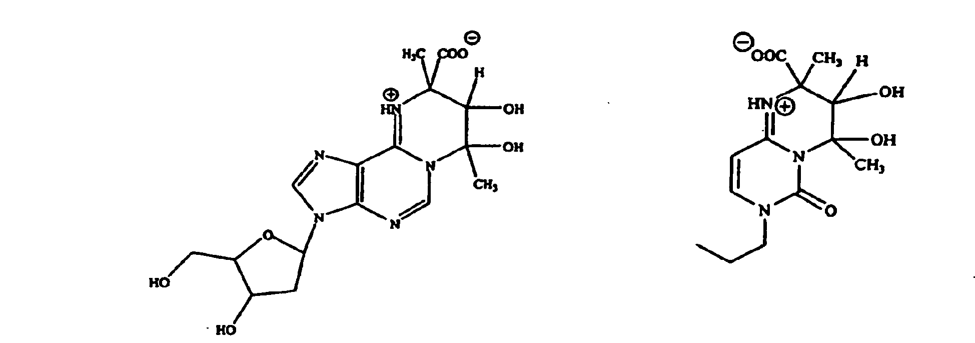

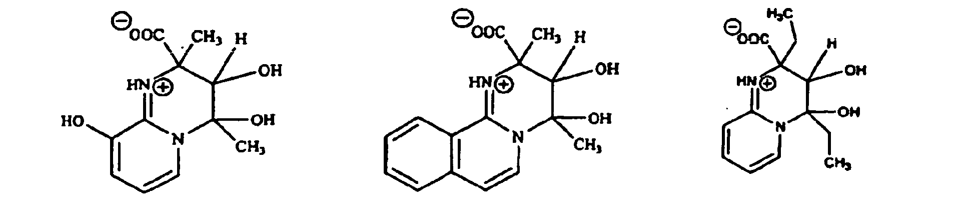

7. Utilisation selon la revendication 2, ***caractérisée* en ce que** la molécule présente la formule suivante : 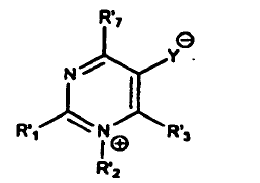 avec R'₁, R'₂, R'₃ et R'₇ représentent : ⁻ , H, ou des chaînes carbonées, substituées ou non, pouvant contenir des hétéroatomes ;
R'₁ et R'₂ pouvant former un cycle aromatique ou non aromatique ;
Y = O ou S

8. Utilisation selon la revendication 7, ***caractérisée* en ce que** la molécule a une formule choisie dans le groupe comprenant :

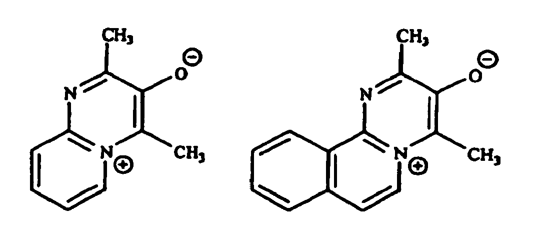

9. Utilisation selon la revendication 2, ***caractérisée* en ce que** la molécule a la formule suivante :

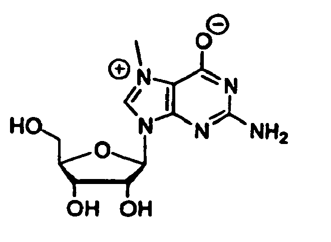

10. Molécule telle que définie à l'une des revendications 3 à 9 pour son utilisation comme médicament.

11. Molécule, se présentant sous la forme d'un zwittérion à pH physiologique, de formule générale: 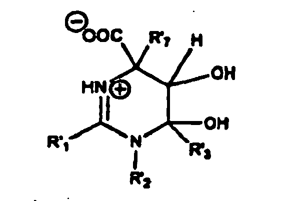 susceptible d'être obtenue selon la réaction : 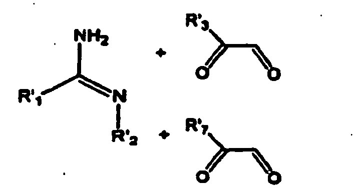 avec R'₁, R'₂, R'₃ et R'₇ représentent H ou des chaînes carbonées, substituées ou non, cycliques ou non, aromatiques ou non, pouvant contenir des hétéroatomes,
et si R'₃=R'₇=CH3, c'est à dire si l'α-cétoaldéhyde est le méthylglyoxal, 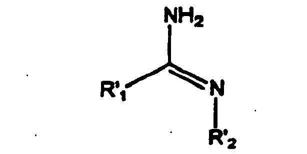 n'est pas la 2-aminopyridine, l'adénine, l'adénosine, la 2'-désoxyadénosine, la 9-propyladénine, la cytosine, l'acide polyadénylique (polyA), l'arginine ou ses dérivés protégés sur le groupe α-amino acide.

12. Molécule, se présentant sous la forme d'un zwittérion à pH physiologique, de formule générale : 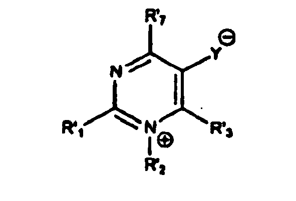 avec R'₁ et R'₂ représentent : ⁻ , H, ou des chaînes carbonées, substituées ou non, pouvant contenir des hétéroatomes ; et pouvent former un cycle aromatique ou non aromatique ;
R'₃ et R'₇ représentent : H, ou des chaînes carbonées, substituées ou non, pouvant contenir des hétéroatomes ;
Y = O ou S.

13. Molécule selon la revendication 12 choisie dans le groupe comprenant : 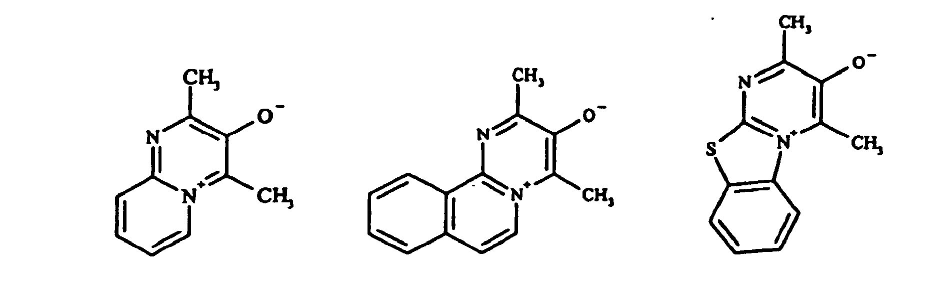 ou leurs dérivés ayant un atome d'oxygène (O) substitué par un atome de soufre (S), par exemple : 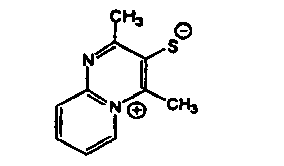 et/ou possédant des groupements R'₃ et/ou R'₇ modifiés, par condensation des groupements méthyles avec un aldéhyde, par exemple :

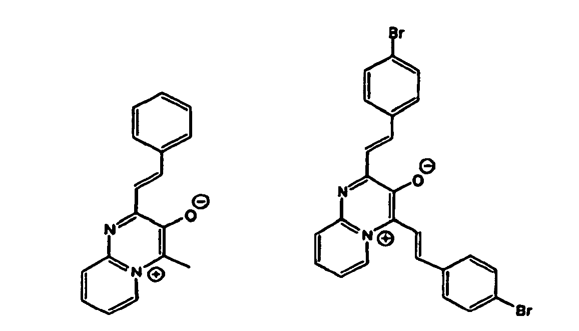

14. Procédé de préparation d'une molécule, objet de l'une des revendications 12 à 13, ***caractérisé* en ce qu'**il comprend les étapes suivantes :
- condensation de 2 molécules d'α-cétoaldéhyde possédant des atomes d'hydrogène énolisables sur une molécule possédant deux atomes d'azote séparés par un carbone capables de réagir avec l'α-cétoaldéhyde, donnant naissance à un α-iminoacide : 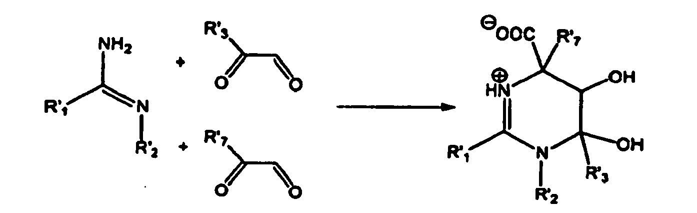
- chauffage de l'α-iminoacide en milieu basique, aboutissant à une décarboxylation oxydante et une déshydratation selon la réaction suivante : 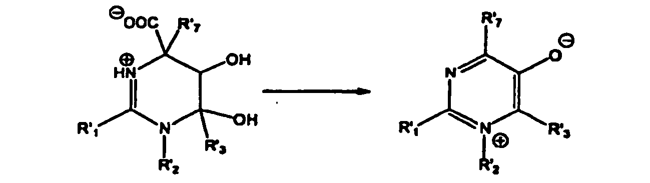
- éventuellement, substitution de l'atome d'oxygène par un atome de soufre et/ou modification des groupements R'₃ et/ou R'₇ par condensation des groupements méthyles avec un aldéhyde.

## Claims

1. Use of a molecule, which comes in the form of a zwitterion at a physiological pH, with the formula: 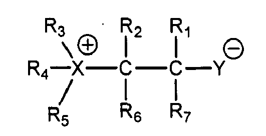 where X = N or P;
Y = O or S;
R₁ to R₇ represent: ⁻ , H, or carbon chains, substituted or not, which may contain heteroatoms;
except for betaine,
for preparing a medicinal product designed to treat diseases included in the group: cystic fibrosis, bronchial restricting diseases, such as asthma, or diarrhoea.

2. Use as claimed in claim 1, ***characterised* in that** R₁ and/or R₂ and/or R₃ and/or R₄ and/or R₅ and/or R₆ and/or R₇ form an aromatic or non-aromatic ring.

3. Use as claimed in claim 2, ***characterised* in that** the molecule has the following formula: 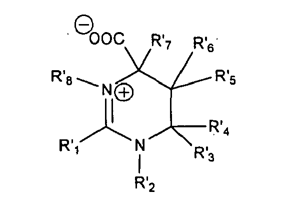 where R'₁ to R'₈ represent: ⁻ , H, or carbon chains, substituted or not, which may contain heteroatoms;
R'₁ and R'₂ may form an aromatic or non-aromatic ring.

4. Use as claimed in claim 3, ***characterised* in that**:
- R'₄ = R'₅ = OH;
- R'₆ = R'₈ = H.

5. Use as claimed in claim 4, ***characterised* in that**:
- R'₃ = R'₇ = CH₃.

6. Use as claimed in claim 5, ***characterised* in that** the molecule has a formula included in the group:

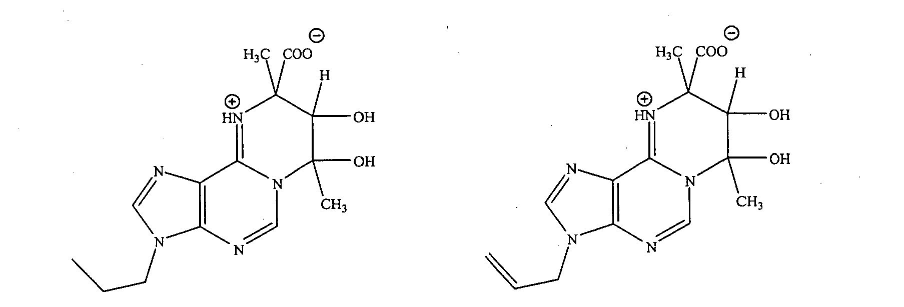

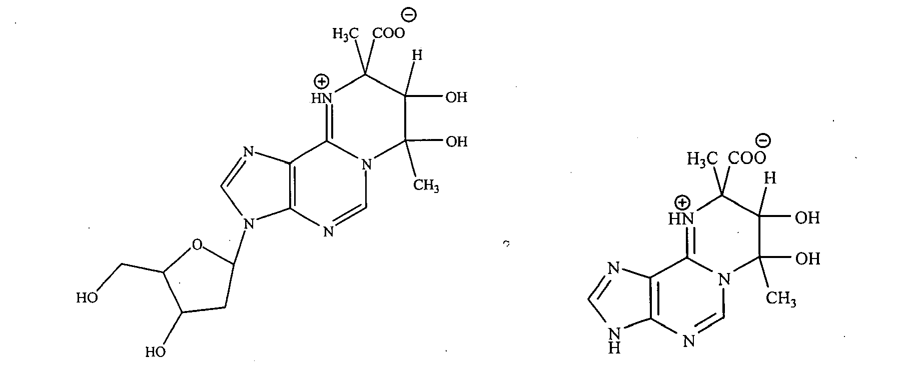

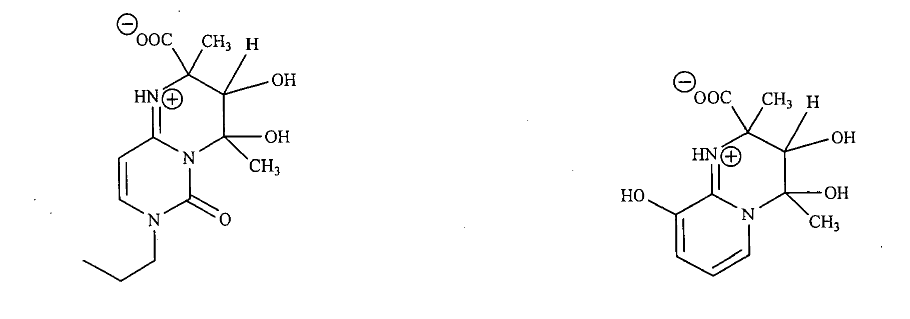

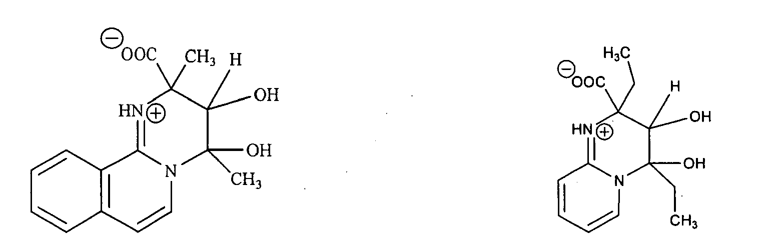

7. Use as claimed in claim 2, ***characterised* in that** the molecule has the following formula: 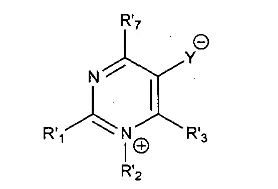 where R'₁, R'₂, R'₃ and R'₇ represent:-, H, or carbon chains, substituted or not, which may contain heteroatoms;
R'₁ and R'₂ may form an aromatic or non-aromatic ring;
Y = O or S

8. Use as claimed in claim 7, ***characterised* in that** the molecule has a formula included in the group:

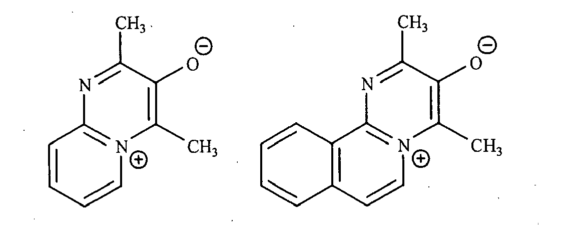

9. Use as claimed in claim 2, ***characterised* in that** the molecule has the following formula:

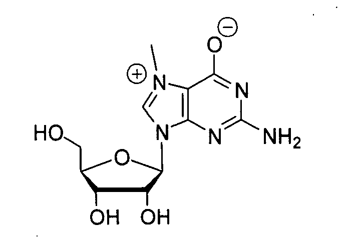

10. A molecule as defined in one of claims 3 to 9 for its use as a medicinal product.

11. A molecule, coming in the form of a zwitterion at physiological pH, with the general formula: 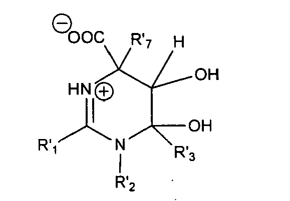 susceptible to be obtained through the reaction: 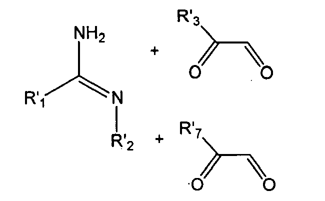 where R'₁, R'₂, R'₃ and R'₇ represent H or carbon chains, substituted or not, cyclic or not, aromatic or not, which may contain heteroatoms,
and if R'₃ = R'₇ = CH3, i.e. if the α-ketoaldehyde is methylglyoxal, 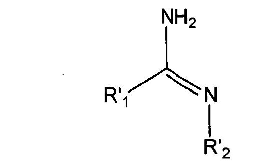 is not 2-aminopyridine, adenine, adenosine, 2'- deoxyadenosine, 9- propyladenine, cytosine, polyadenylic acid (polyA), arginine or its protected derivatives on the α-amino acid group.

12. A molecule, coming in the form of a zwitterion at physiological pH, with the general formula: 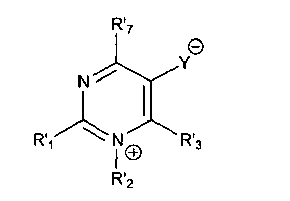 where R'₁ and R'₂ represent: ⁻ , H, or carbon chains, substituted or not, which may contain heteroatoms; and which may form an aromatic or non-aromatic ring;
R'₃ and R'₇ represent: H, or carbon chains, substituted or not, which may contain heteroatoms;
Y = O or S.

13. A molecule as claimed in claim 12 included in the group: 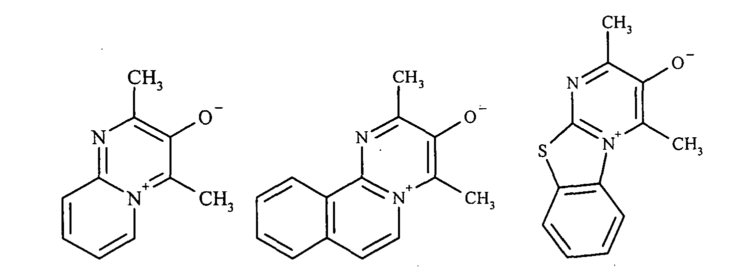 or their derivatives having an oxygen atom (O) substituted by a sulphur atom (S), for example: 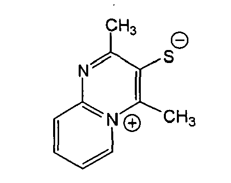 and/or having R'₃ and/or R'₇ groups modified by condensation of the methyl groups with an aldehyde, for example:

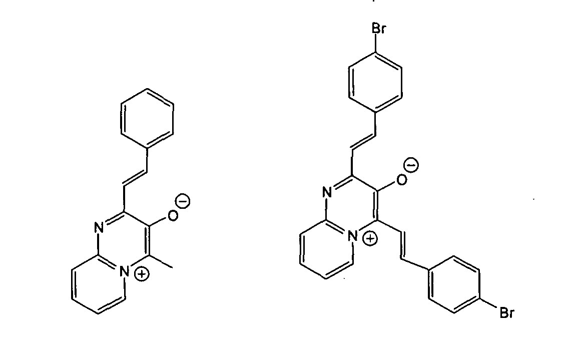

14. Method for preparing a molecule, subject of one of claims 12 or 13, ***characterised* in that** it includes the following steps:
- condensation of two α-ketoaldehyde molecules having enolisable hydrogen atoms on a molecule having two nitrogen atoms separated by a carbon capable of reacting with the α-ketoaldehyde, giving rise to an α-iminoacid: 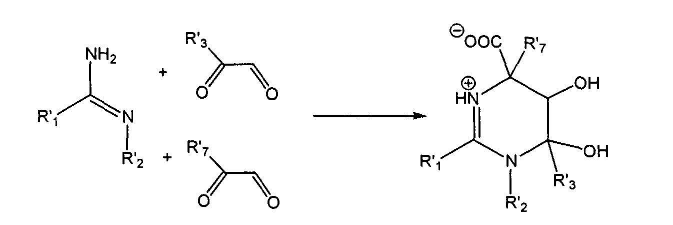
- heating the α-iminoacid in a base medium, leading to oxidising decarboxylation and dehydration according to the following reaction: 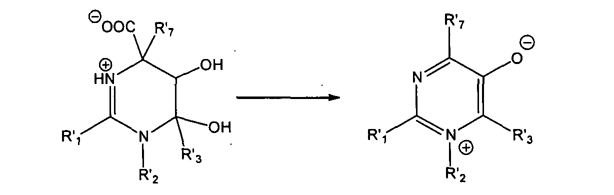
- possibly, substitution of the oxygen atom by a sulphur atom and/or modification of the R'₃ and/or R'₇ groups by condensation of the methyl groups with an aldehyde.

## Patentansprüche

1. Verwendung eines Moleküls, das bei einem physiologischen pH-Wert in der Form eines Zwitterions vorliegt, mit der Formel: 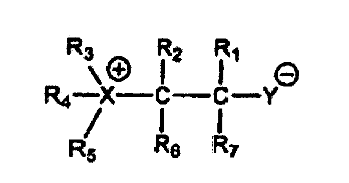 worin X = N oder P;
Y = 0 oder S;
R₁ bis R₇ darstellen: -, H oder substituierte oder nicht substituierte Kohlenstoffketten, die Heteroatome enthalten können;
mit Ausnahme von Betain,
für die Herstellung eines Medikaments, das für die Behandlung von Krankheiten bestimmt ist, die aus der Gruppe ausgewählt sind, umfassend: Mukoviszidose, Bronchien verengende Krankheiten, wie Asthma, oder Durchfall.

2. Verwendung nach Anspruch 1, **dadurch gekennzeichnet, dass** R₁ und/oder R₂ und/oder R₃ und/oder R₄ und/oder R₅ und/oder R₆ und/oder R₇ einen aromatischen oder nicht aromatischen Ring bilden.

3. Verwendung nach Anspruch 2, **dadurch gekennzeichnet, dass** das Molekül die folgende Formel aufweist: 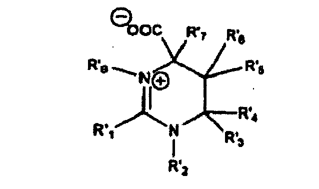 worin R'₁ bis R'₈ darstellt: -, H oder substituierte oder nicht substituierte Kohlenstoffketten, die Heteroatome enthalten können;
wobei R'₁ und R'₂ einen aromatischen oder nicht aromatischen Ring bilden können.

4. Verwendung nach Anspruch 3, **dadurch gekennzeichnet, dass**:
- R'₄ = R'₅ = OH;
- R'₆ = R'₈ = H.

5. Verwendung nach Anspruch 4, **dadurch gekennzeichnet, dass**
- R'₃ = R'₇ = CH₃.

6. Verwendung nach Anspruch 5, **dadurch gekennzeichnet, dass** das Molekül eine Formel besitzt, die aus der Gruppe ausgewählt ist, die umfasst:

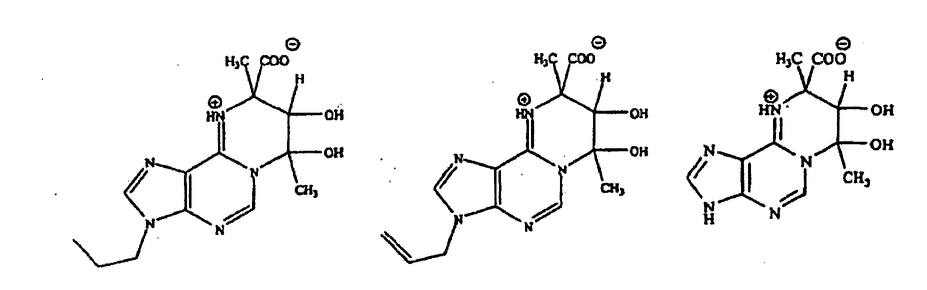

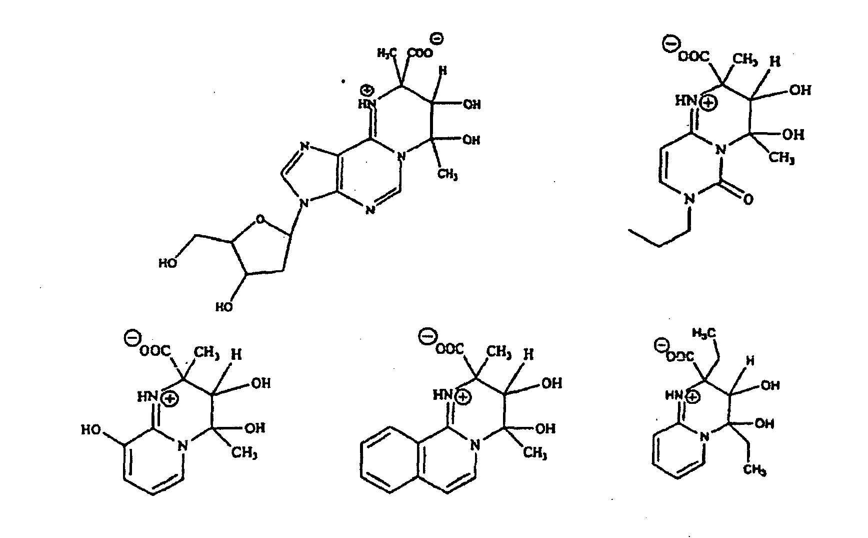

7. Verwendung nach Anspruch 2, **dadurch gekennzeichnet, dass** das Molekül die folgende Formel aufweist: 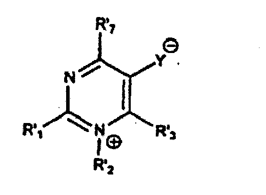 worin R'₁, R'₂, R'₃ und R'₇ darstellen: -, H oder substituierte oder nicht substituierte Kohlenstoffketten, die Heteroatome enthalten können;
wobei R'₁ und R'₂ einen aromatischen oder nicht aromatischen Ring bilden können;
Y = O oder S.

8. Verwendung nach Anspruch 7, **dadurch gekennzeichnet, dass** das Molekül eine Formel aufweist, die aus der Gruppe ausgewählt ist, die umfasst:

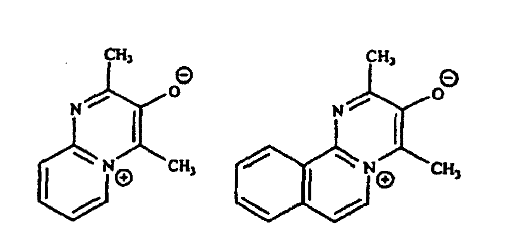

9. Verwendung nach Anspruch 2, **dadurch gekennzeichnet, dass** das Molekül die folgende Formel aufweist:

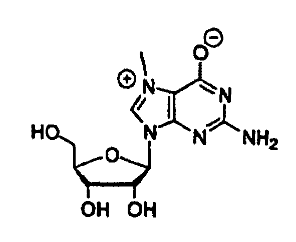

10. Molekül, wie es in einem der Ansprüche 3 bis 9 definiert ist, für seine Verwendung als Medikament.

11. Molekül, das in der Form eines Zwitterions mit einem physiologischen pH-Wert vorliegt, der allgemeinen Formel: 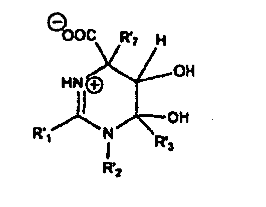 das gemäß der Reaktion erhalten werden kann: 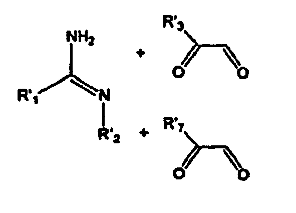 worin R'₁, R'₂, R'₃ und R'₇ H oder substituierte oder nicht substituierte, zyklische oder nicht zyklische, aromatische oder nicht aromatische Kohlenstoffketten darstellen, die Heteroatome enthalten können,
und, wenn R'₃ = R'₇ = CH3, d.h. wenn das α-Cetoaldehyd Methylglyoxal ist, 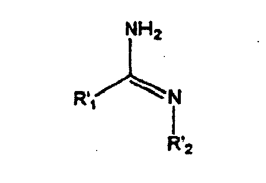 nicht 2-Aminopyridin, Adenin, Adenosin, 2'-Desoxyadenosin, 9-Propyladenin, Cytosin, Polyadenylsäure (PolyA), Arginin oder seine an der α-Aminosäuregruppe geschützten Derivate ist.

12. Molekül, das bei einem physiologischen pH-Wert in der Form eines Zwitterions vorliegt, der allgemeinen Formel: 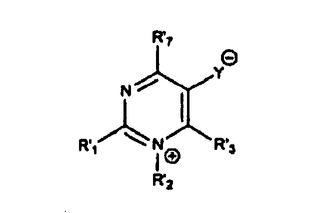 worin R'₁ und R'₂ darstellen: -, H oder substituierte oder nicht substituierte Kohlenstoffketten, die Heteroatome enthalten können; und einen aromatischen oder nicht aromatischen Ring bilden können;
R'₃ und R'₇ darstellen: H oder substituierte oder nicht substituierte Kohlenstoffketten, die Heteroatome enthalten können;
Y = O oder S.

13. Molekül nach Anspruch 12, das aus der Gruppe ausgewählt ist, die umfasst: 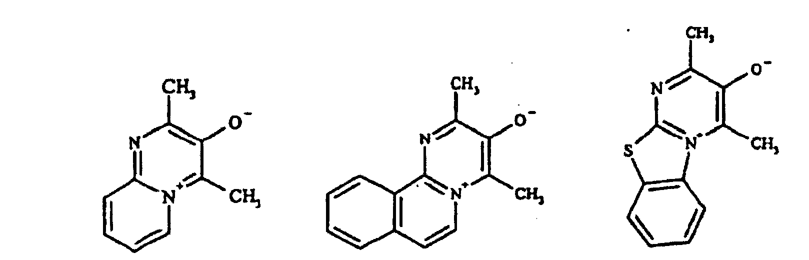 oder ihre Derivate, die ein Sauerstoffatom (O) aufweisen, das durch ein Schwefelatom (S) substituiert ist, beispielsweise: 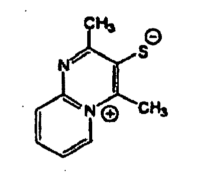 und/oder Gruppen R'₃ und/oder R'₇ besitzen, die durch Kondensation der Methylgruppen mit einem Aldehyd modifiziert sind, beispielsweise:

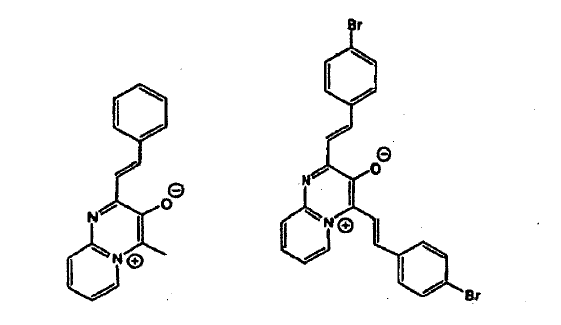

14. Verfahren zur Herstellung eines Moleküls, Gegenstand eines der Ansprüche 12 bis 13,
**dadurch gekennzeichnet, dass** es die folgenden Schritte umfasst:
- Kondensation von 2 α-Cetoaldehydmolekülen, die enolisierbare Wasserstoffatome besitzen, an einem Molekül, das zwei durch einen Kohlenstoff getrennte Stickstoffatome besitzt, die mit α-Cetoaldehyd reagieren können, was eine α-Aminosäure ergibt: 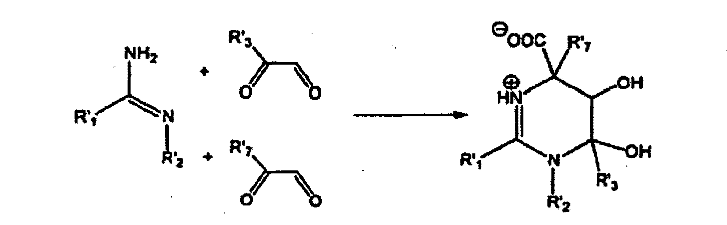
- Erhitzen der α-Iminosäure in basischem Medium, was zu einer oxidierenden Decarboxylierung und Dehydratisierung nach der folgenden Reaktion führt: 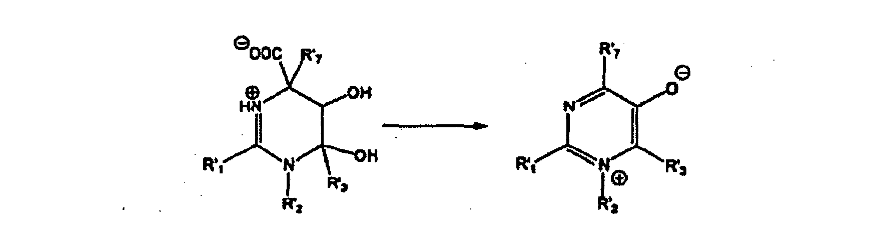
- gegebenenfalls Substitution des Sauerstoffatoms mit einem Schwefelatom und/oder Modifizierung der Gruppen R'₃ und/oder R'₇ durch Kondensation der Methylgruppen mit einem Aldehyd.
